# EUROPÄISCHE PATENTSCHRIFT

(11) **EP 2 359 904 B1**
(45) Veröffentlichungstag und Bekanntmachung des Hinweises auf die Patenterteilung: **07.12.2011**
(21) Anmeldenummer: 10153450.1
(22) Anmeldetag: 12.02.2010
(51) Int. Cl.: A61N 5/10

(54) **Magazin für Kettenbestandteile einer Kette mit radioaktiven Strahlenquellen sowie ein System aus einem Kettenbestandteil und einem Magazin**
Cartridge for chain components of a chain with radioactive radiation sources and a system comprising a chain component and a cartridge
Cartouche pour composants de chaînes d'une chaîne dotée de sources de rayonnement radioactives ainsi qu'un système composé d'un composant de chaîne et d'une cartouche

(43) Veröffentlichungstag der Anmeldung: 24.08.2011
(73) Patentinhaber: Eckert & Ziegler Bebig GmbH, 13125 Berlin (DE)
(72) Erfinder: Hentrich, Axel, 13125, Berlin (DE); Lederer, Christoph, 10437, Berlin (DE)
(74) Vertreter: Gulde Hengelhaupt Ziebig & Schneider

(56) Entgegenhaltungen:
- US-A1- 2002 032 360
- US-A1- 2002 193 656
- US-A1- 2004 077 919
- US-A1- 2005 267 319
- US-B1- 6 837 844

## Beschreibung

Die Erfindung betrifft ein Magazin für Kettenbestandteile einer Kette mit radioaktiven Strahlenquellen sowie ein System aus Kettenbestandteilen und einem Magazin für Kettenbestandteile einer Kette mit radioaktiven Strahlenquellen. Die Verwendung des Magazins ist beispielsweise für die Herstellung von Ketten aus Strahlenquellen und Abstandshaltern zur Behandlung von Prostatakarzinomen in der Brachytherapie konzipiert.

### Stand der Technik

Es ist bekannt, Tumorpatienten, insbesondere Prostatatumorpatienten eine Kette bestehend aus radioaktiven Strahlenquellen, sogenannten Seeds einzupflanzen. Dazu sind sowohl Einzelstrahlenquellen als auch eine vorkonfigurierte Seedketten aus z.B. abwechselnd einer radioaktivem Strahlenquelle und einem nichtaktiven Abstandshalter oder Spacer bekannt. Die Implantation von Einzelstrahlenquellen erlaubt unter Verwendung des entsprechenden Instrumentariums eine individuelle Positionierung der einzelnen Strahlenquellen. Bedingt durch die entzündliche Vergrößerung der Prostata nach der Implantation gefolgt von einem Abschwellen kann es jedoch unter Umständen zu einer Migration der Strahlenquellen bzw. zu einer Verschiebung ihrer planbestimmten Position kommen. Eine Strahlenquellenkette verhindert diese Positionsverschiebung, da sie die Einzelimplantate miteinander verbindet.

Neue medizinische Untersuchungen zeigen, dass die Einhaltung eines individuell auf den Patienten abgestimmter Bestrahlungsplanes die besten Resultate bei der Tumorbehandlung erzielt. Diesen einzuhalten erfordert die auf jeden Patienten angepasste genaue Positionierung der einzelnen radioaktiven Strahlenquellen.

Aus WO 2009/005528 A1 ist ein Magazin und ein Gerät zum Zusammenfügen von frei konfigurierbaren Seed-Spacer-Ketten aus insgesamt fünf verschiedenen Magazinen mit nebeneinander angeordneten Implantaten bekannt. Die Magazine führen die übereinandergestapelten Kettenbestandteile linear nach unten aus dem Magazin heraus. Diese Anordnung ist jedoch unvorteilhaft, da mit zunehmender Anzahl der Kettenbestandteile die Magazine unhandlich groß werden. Die Magazine bestehen dabei aus transparentem Polymer und sind daher nur in der Verpackung bzw. nach Einstecken in das Beladungsgerät strahlensicher. Der Inhalt der Magazine ist auch aus diesen Gründen auf ca. 20 Strahlenquellen begrenzt. Somit müssen die Magazine bei einer Konfektionierung mehrerer Ketten gewechselt werden. US 6,726,617 B1 beschreibt ebenso lineare Magazine für die Brachytherapie. In US 6,648,811 B2 und US 6,572,527 B2 werden die Kettenbestandteile linear hintereinander angeordnet. Auch dies hat den Nachteil, dass die Magazine nicht platzsparend gelagert werden können.

US 6,616,593 B1 beschreibt ein automatisiertes kreisförmiges Strahlenquellenmagazin, in dem verschiedene Kettenbestandteile wie Strahlungsquellen und Abstandshalter zusammen in unterschiedlichen Kammern geführt werden. Der Antrieb und Auswurf der Kettenbestandteile im Magazin erfolgt dabei elektrisch. Die Magazine sind somit auf einen externen Antrieb angewiesen, ein interner Vortriebsmechanismus existiert nicht. Dies hat den Nachteil, dass ein solches Magazin nicht vollständig sterilisiert werden kann. Zudem muss das Magazin ersetzt werden, wenn z.B. der Abstandshaltervorrat erschöpft ist, jedoch der Strahlenquellenvorrat noch nicht. Somit könnte der Bediener einer Strahlung beim Ersetzen des Magazins ausgesetzt werden.

US 6,454,696 B1 beschreibt direkt hintereinanderliegende Rundmagazine, in denen die Strahlenquellen und Abstandshalter konzentrisch zum Drehpunkt der Magazine angeordnet sind. Die Magazine werden wiederum von außen angetrieben. Die Implantate werden nicht aus dem Magazin herausgeworfen, sondern in ein Öffnung im Inneren des Magazins platziert und von dort durch einen externen Draht aus dem Magazin herausbefördert. Abstandshalter und Strahlenquellenmagazin sind dabei nachteilig so miteinander verbunden, dass die Einzelimplantate des hinteren Magazins konstruktionsbedingt durch das vordere Magazin durchgeschoben werden müssen. Das Hindurchschieben durch verschiedene Magazine führt jedoch häufig zu einem Verklemmen der Vorrichtung, welches dann manuell wieder beseitigt werden muss. Ein Hindurchschieben von Kettenbestandteilen durch verschiedene Magazine ist auch aus US 6,358,195 B1 bekannt.

US 2002/0032360 A1 offenbart ein Magazin in dem Strahlenkettenbestandteile sowohl radial als auch linear hintereinander gelagert werden und über einen Auswurf ausgegeben werden können. Schieber werden dabei verwendet, um die Kettenbestandteile nach außen zu befördern.

### Offenbarung der Erfindung

Aufgabe der Erfindung ist es daher, ein Magazin und ein System für Kettenbestandteile einer Kette mit radioaktiven Strahlenquellen bereitzustellen, welche die Nachteile des Standes der Technik überwindet.

Eine Aufgabe ist dabei, ein Magazin anzugeben, welches vollsterilisierbar ist. Ferner ist es Aufgabe, die Kettenbestandteile platzsparend in dem Magazin zu lagern. Es soll dem behandelnden OP-Personal die Möglichkeit geben werden, unter Einhaltung eines individuellen Bestrahlungsplanes die Seedimplantation anwenderfreundlich durchzuführen. Anwenderfreundlich ist hierbei bezogen auf die Magazinbedienung sowie auf den optimalen Strahlenschutz für den Anwender zu verstehen.

Es wird daher ein Magazin für Kettenbestandteile einer Kette mit radioaktiven Strahlenquellen vorgeschlagen, welches ein Gehäuse umfasst sowie ein erstes Mittel zur Aufnahme von Kettenbestandteilen, das fest in dem Gehäuse gelagert ist und Vertiefungen zur Aufnahme von Kettenbestandteilen aufweist, sowie einen Auswurf zum Auswurf der Kettenbestandteile. Ferner ist in dem Gehäuse eine Spannfeder zum Vortrieb des Mittels zur Aufnahme von Kettenbestandteilen angeordnet, einen Zahnkranz, der fest mit dem Mittel zur Aufnahme von Kettenbestandteilen gekoppelt und drehbar in dem Gehäuse angeordnet ist, und einen Auswurf zum Auswurf der Kettenbestandteile. Ferner umfasst das Magazin einen Auswerferhebel, der rotatorisch so in dem Gehäuse gelagert ist, dass er in einer ersten Stellung in den Zahnkranz eingreift und in einer zweiten Stellung den Auswurf blockiert.

Vorteilhafterweise benötigt ein erfindungsgemäßes Magazin keine elektromechanischen Bauteile zum Vortrieb der Kettenbestandteile, da es einen eigenen Vortrieb in Form einer Spannfeder aufweist.

Bevorzugt sind der Zahnkranz und die Spannfeder einstückig ausgebildet. Sie können durch Spritzgießen einfach und kostengünstig hergestellt werden.

In einem bevorzugten Ausführungsbeispiel ist genau ein Auswurf vorgesehen, wodurch eine Strahlenbelastung minimiert werden kann.

Das Magazin ist bevorzugt für genau eine Art von Kettenbestandteilen konfiguriert. Dass heißt, es nimmt entweder Strahlenquellen oder Abstandshalter auf, aber bevorzugt nicht beide zusammen.

Das Magazin umfasst ferner bevorzugt einen Verschlussschieber zum Öffnen und Verschließen des Auswurfs, der verschiebbar in dem Gehäuse gelagert ist. Der Verschlussschieber ermöglicht es weiter, eine Strahlenbelastung zu minimieren. Es ist besonders bevorzugt translatorisch verschiebbar ausgebildet.

Das Mittel zur Aufnahme von Kettenbestandteilen ist dabei bevorzugt kreisförmig ausgebildet. Ebenso ist der Zahnkranz bevorzugt kreisförmig ausgebildet. Die Vertiefungen sind in einem Ausführungsbeispiel auf dem Umfang des kreisförmigen Mittels zur Aufnahme von Kettenbestandteilen angeordnet. Die Lagerung auf dem Umfang ermöglicht die Aufnahme einer großen Anzahl von Kettenbestandteilen.

Es kann ferner eine Begrenzung um das Mittel zur Aufnahme von Kettenbestandteilen herum im Gehäuse ausgebildet sein. Die Größe des Abstandes von Begrenzung und dem Mittel zur Aufnahme von Kettenbestandteilen ist bevorzugt derart ausgebildet, dass Kettenbestandteile zwischen dem Mittel zur Aufnahme von Kettenbestandteilen und der Begrenzung im Inneren des Gehäuses lagerbar und führbar sind. Dies bedeutet, dass die Kettenbestandteile auf dem Umfang des Mittels zur Aufnahme von Kettenbestandteilen zum Auswurf geführt werden können, ohne dass sie herausfallen. Diese Anordnung ermöglicht eine platzsparende Lagerung von Kettenbestandteile und die Bereitstellung von Magazinen mit bis zu 100 oder mehr Kettenbestandteilen.

Die Spannfeder ist bevorzugt eine Konstantkraftfeder. Dies ermöglicht einen Vortrieb des Mittels zur Aufnahme von Kettenbestandteilen mit immer derselben Kraft.

Das Gehäuse kann einen kreisförmigen inneren Lagerring aufweisen, an dem die Konstantkraftfeder sowie das Mittel zur Aufnahme von Kettenbestandteilen und der darüber liegende Zahnkranz gelagert sind. Die Konstantkraftfeder ist bevorzugt unter dem Mittel zur Aufnahme von Kettenbestandteilen und der Zahnkranz über dem Mittel zur Aufnahme von Kettenbestandteilen angeordnet und miteinander verbunden, so dass die Konstantkraftfeder die Aufnahme von Kettenbestandteilen vortreibt.

Der Bereich des Mittels zur Aufnahme von Kettenbestandteilen, der am inneren Lagerring des Gehäuses anliegt, kann von außen betrachtbar ausgebildet sein und mit mindestens einer Markierung zur Inhaltsanzeige versehen sein. Dieser anliegende Bereich ist bevorzugt entsprechend ringförmig ausgestaltet.

Am Gehäuse können Sicherungsnuten zum Eingriff von Sicherungselementen zur Sicherung des Magazins in einem Gerät zur Konfektionierung von Kettenbestandteilen angeordnet sein.

Das Gehäuse umfasst bevorzugt eine Kodierungsöffnung, die mit dem Verschlussschieber derart zusammenwirkt, dass bei Einführung eines entsprechenden Kodierungselementes in die Kodierungsöffnung der Verschlussschieber den Auswurf freigibt. Durch die beschriebene Kodierung ist es unmöglich, das Strahtenquellen- bzw. das Abstandshaltermagazin beim Einsatz in ein Gerät zum Konfektionieren zu vertauschen.

Der Auswerferhebel ist in einem bevorzugten Ausführungsbeispiel über eine erste Druckfeder und der Verschlussschieber über eine zweite Druckfeder verspannt. Der Auswerferhebel ist über eine zweite Öffnung im Gehäuse von außen betätigbar.

Der Auswurf ist bevorzugt an der Peripherie des Magazins so angeordnet, dass die Kettenbestandteile aus dem Magazin heraus vom Magazin weg auswerfbar sind. In einem Ausführungsbeispiel ist der Auswurf an einer unteren Stirnseite des Magazins angeordnet. Dadurch werden die Kettenbestandteile nach unten hin ausgegeben. Ein Hindurchführen von Kettenbestandteilen durch ein Magazin von außen ist nicht nötig und nicht möglich.

Im Gehäuse ist bevorzugt ein Mittel zum Blockieren des Weiterdrehens des Mittels zur Aufnahme von Kettenbestandteilen nach Auswurf eines Kettenbestandteils realisiert. Dies ermöglicht es, immer nur genau ein Kettenglied auszuwerfen. Die Blockierungsfunktion wird bevorzugt z.T. von dem Zahnkranz in Verbindung mit dem Auswerferhebel übernommen.

Das Gehäuse besteht bevorzugt aus einem strahlungsabsorbierbaren Material, besonders bevorzugt aus Edelstahl. Dadurch kann die Strahlenbelastung weiter minimiert werden.

Ferner wird ein System aus Kettenbestandteilen für eine Kette mit radioaktiven Strahlenquellen und einem erfindungsgemäßen Magazin vorgeschlagen, wobei ein Mittel zum Blockieren des Weiterdrehens des Mittels zur Aufnahme von Kettenbestandteilen nach Auswurf eines Kettenbestandteils durch ein Zusammenwirken von dem dem ausgeworfenen Kettenbestandteil nachfolgendem Kettenbestandteil und dem Auswerferhebel erfolgt. Somit können also die Kettenbestandteile selbst zur Blockade eingesetzt werden.

Der Auswerferhebel blockiert bevorzugt in besagter zweiter Stellung den Auswurf und der dem Auswurf nächste Kettenbestandteil drückt gegen den Auswerferhebel, so dass ein Weiterdrehen des Mittels zur Aufnahme von Kettenbestandteilen blockiert wird. Entsprechend wird ein Verfahren zu Blockade des Vortriebs vorgeschlagen.

### Kurze Beschreibung der Zeichnungen

Ausführungsbeispiele der Erfindung werden anhand der Zeichnungen und der nachfolgenden Beschreibung näher erläutert. Es zeigen:
- Fig. 1: eine Draufsicht auf ein erfindungsgemäßes Magazin für Kettenbestandteile einer Kette mit radioaktiven Strahlenquellen,
- Fig. 2: eine Draufsicht auf ein erfindungsgemäßes Magazin für Kettenbestandteile einer Kette mit radioaktiven Strahlenquellen ohne Deckel,
- Fig. 3: eine Draufsicht auf ein erfindungsgemäßes Magazin für Kettenbestandteile einer Kette mit radioaktiven Strahlenquellen ohne Deckel, ohne Mittel zur Aufnahme von Kettenbestandteilen und ohne Zahnkranz,
- Fig. 4: Kernbestandteile des Magazins ohne Gehäuse,
- Fig. 5: einen vertikalen Schnitt durch das Magazin der Fig. 1,
- Fig. 6-11: den erfindungsgemäßen Auswerfmechanismus in verschiedenen Schritten,
- Fig. 12: den erfindungsgemäßen Blockiermechanismus in den Magazinen
- Fig. 13: zusammenfügbare Abstandshalter und Strahlenquellen,
- Fig. 14: eine perspektivische Ansicht eines erfindungsgemäßen Gerätes zum Fügen und Konfektionieren von Ketten, die Strahlenquellen beinhalten;
- Fig. 15: eine Seitenansicht des erfindungsgemäßen Gerätes zum Fügen und Konfektionieren von Ketten der Fig. 14,
- Fig. 16: einen perspektivischen Querschnitt durch das erfindungsgemäße Gerät entlang der Achse A -A in Fig. 15, gesehen entgegen der Richtung der Pfeile der Fig. 15,
- Fig. 17: einen Querschnitt durch das erfindungsgemäße Gerät entlang der Achse A-A in Fig. 15, gesehen in Richtung der Pfeile der Fig. 15,
- Fig. 18: eine erste Ansicht des erfindungsgemäßen Gerätes ohne Gehäuse,
- Fig. 19: eine zweite Ansicht des erfindungsgemäßen Gerätes ohne Gehäuse,
- Fig. 20: einen Querschnitt durch das Gerät zum Fügen durch ein Abstandshaltermagazin;
- Fig. 21: eine Vergrößerung der Fig. 20 im unteren Bereich des Abstandshaltermagazins;
- Fig. 22: einen Längsschnitt durch den vorderen Bereich des erfindungsgemäßen Gerätes zum Fügen im Bereich des Nadelhalters.

### Detaillierte Beschreibung der Zeichnungen

Die vorliegende Erfindung beschreibt ein Magazin und ein System für Kettenbestandteile einer Kette, die radioaktive Strahlenquellen umfasst. Die Verwendung des Magazins und des Systems ist für die Herstellung von Ketten aus Strahlenquellen und Abstandshaltern zur Behandlung von Prostatakarzinomen in der Brachytherapie konzipiert. Es kann aber auch für andere Anwendungen, wie z.B. die Herstellung von Ketten mit Strahlenquellen zur Behandlung von Mammalkarzinomen verwendet werden.

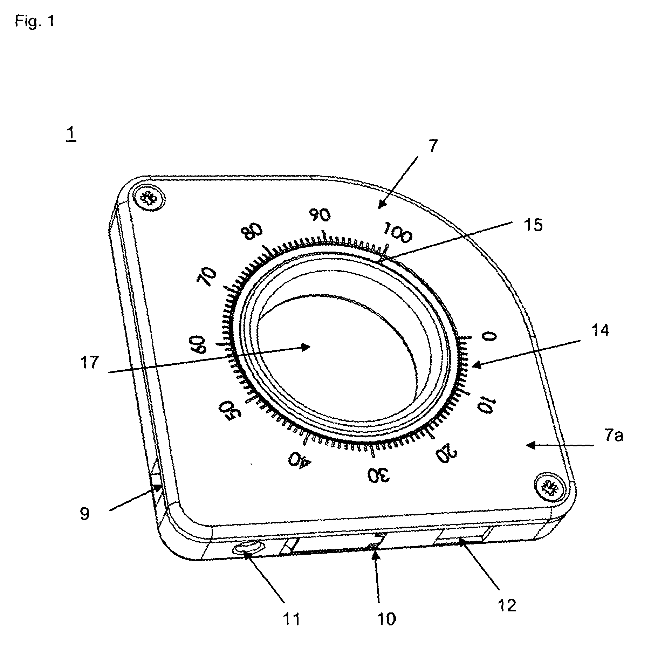

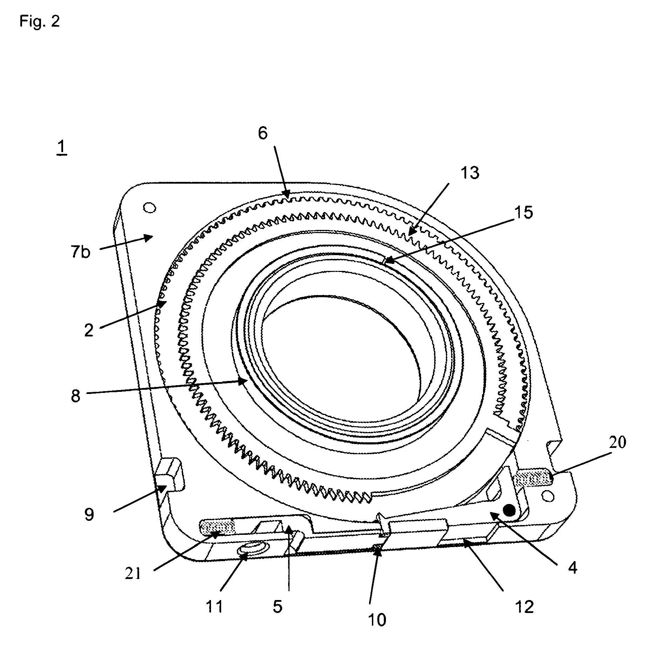

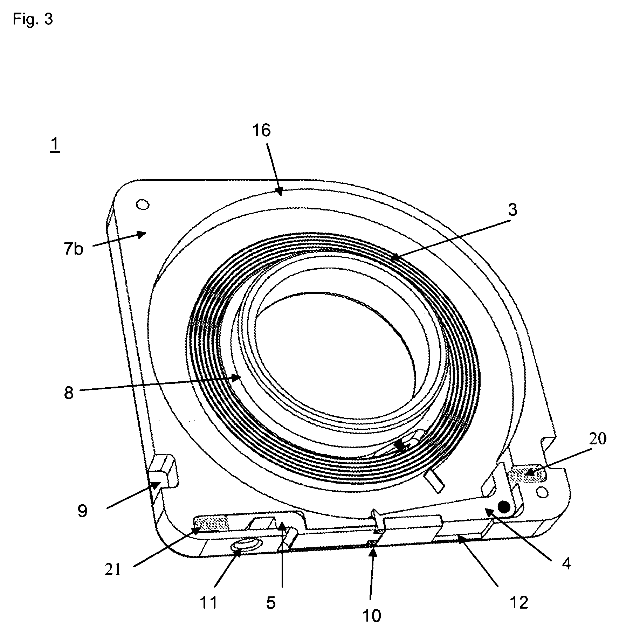

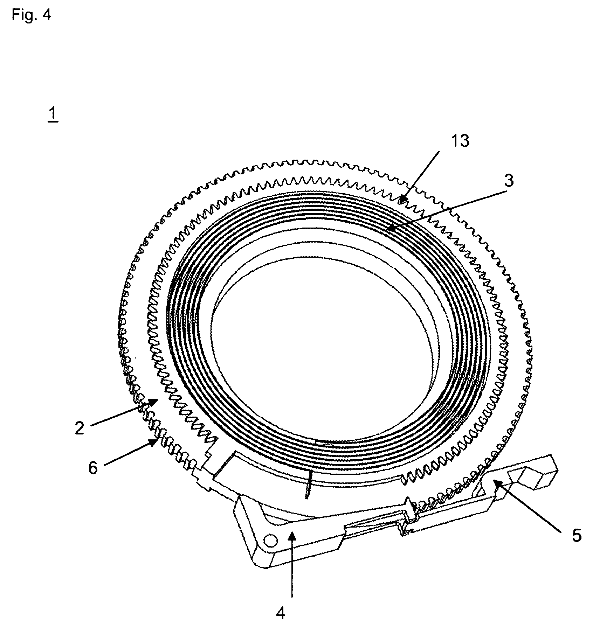

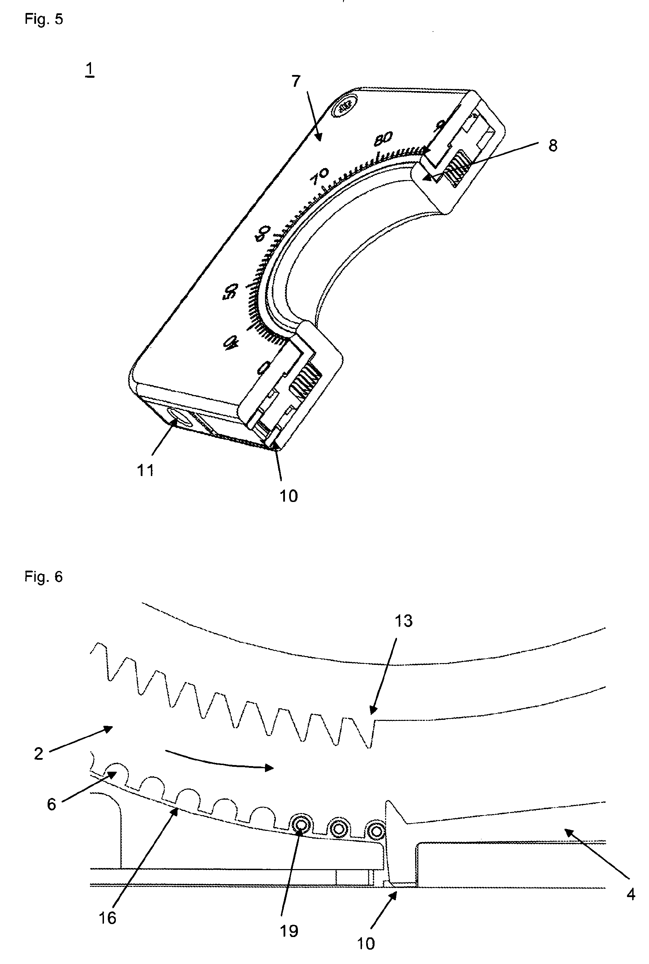

Fig. 1 zeigt eine Draufsicht auf ein erfindungsgemäße Magazin 1 für Kettenbestandteile einer Kette mit radioaktiven Strahlenquellen. In Fig. 2 ist ein Magazin ohne Deckel 7a gezeigt. In Fig. 3 fehlen zudem ein Mittel zur Aufnahme von Kettenbestandteilen 2 und ein Zahnkranz 13. Fig. 4 zeigt die Kernbestandteile des Magazins ohne Gehäuse 7 und Fig. 5 zeigt einen vertikalen Schnitt durch das Magazin der Fig. 1.

Wie in Fig. 1 und 2 gezeigt, umfasst ein erfindungsgemäßes Magazin 1 ein Gehäuse 7 bestehend aus einem Deckel 7a und einer Gehäuseschale 7b. Der Deckel umfasst eine Anzeige 14 zum Anzeigen des Füllstandes des Magazins in Verbindung mit einer Markierung 15. Die Markierung ist bevorzugt auf einem inneren Lagerring des Mittels zur Aufnahme von Kettenbestandteilen 2 angeordnet.

An der Peripherie des Magazins 1, bevorzugt an der unteren Seitenwand des Gehäuses 7, ist eine Kodierungsbohrung 11, ein Auswurf 10 und eine erste Öffnung 12 angeordnet. Es ist bevorzugt nur genau ein Auswurf bzw. eine Auswurfsöffnung 10 vorgesehen, damit die Strahlenbelastung bei Verwendung von Strahlenquellen als Kettenbestandteile minimal gehalten werden kann. Der Auswurf erfolgt also in derselben oder einer parallelen Ebene, in der sich das Mittel zur Aufnahme von Kettenbestandteilen dreht. Der Auswurf des Kettenbestandteils erfolgt vom Magazin weg. Hat der Kettenbestandteil den Auswurf verlassen, so besteht kein Kontakt mehr zwischen Magazin und Kettenbestandteil. Kodierungsbohrung 11 und/oder Auswurf 10 und/oder erste Öffnung 12 sind bevorzugt getrennt vom Mittel zur Aufnahme von Kettenbestandteilen 2 unterhalb des Mittels zur Aufnahme von Kettenbestandteilen 2 aber in derselben oder eine parallelen Ebene angeordnet.

In einem bevorzugten Ausführungsbeispiel ist das Magazin nur für genau eine Sorte von Kettenbestandteilen konfiguriert, also entweder für Strahlenquellen oder für Abstandshalter. Somit kann keine Verwechslung der ausgeworfenen Kettenbestandteile auftreten.

In Verbindung mit der ersten Öffnung 12 ist ein Auswerferhebel 4 angeordnet, der drehbar im Gehäuse 7 gelagert ist. Der Auswerferhebel 4 ist durch die erste Öffnung 12 von außen betätigbar. In Ruhestellung verschließt der Auswerferhebel 4 den Auswurf 10 von innen. Der Auswurf 10 steht ferner mit einem Verschlussschieber 5 in Verbindung, der durch die Kraft einer zweiten Spannfeder 21 den Auswurf 10 nach außen verschließt. Der Verschlussschieber 5 ist translatorisch verschiebbar, so dass der Auswurf 10 bei Betätigung des Verschlussschiebers 5 freigegeben werden kann. Der Verschlussschieber 5 ist über eine Öffnung 11 oder Kodierungsbohrung 11 zur Freigabe des Auswurfs 10 betätigbar. Ferner weist das Magazin bevorzugt aber nicht beschränkend eine zentrale Öffnung 17 auf. Wie man in Fig. 2 sehen kann, ist in der hinteren Gehäuseschale 7b ein kreisförmiges Mittel zur Aufnahme von Kettenbestandteilen 2, auch im Falle eines Magazins für Strahlenquellen "Seedablage" 2 genannt, drehbar gelagert eingesetzt. Das Mittel zur Aufnahme von Kettenbestandteilen 2 umfasst dabei radial angeordnete Vertiefungen 6, die derart ausgebildet sind, dass sie Kettenbestandteile wie Strahlenquellen 19 oder Abstandshalter 18 aufnehmen und gegen ein Herausfallen lagern können. Die Vertiefungen 6 sind bevorzugt der Form der Kettenbestandteile entsprechend ausgebildet. Besonders bevorzugt sind sie halbkreisförmig auf dem kreisförmigen Rand entlang des Umfangs des Mittels zur Aufnahme von Kettenbestandteilen 2 angeordnet. Mit anderen Worten, dass Mittel zur Aufnahme von Kettenbestandteilen 2 ist einem Zahnrad ähnelnd ausgebildet, umfasst aber anstatt von Zähnen Vertiefungen 6 auf seinem äußeren Rand. Eine Spannfeder 3, bevorzugt eine Konstantkraftfeder 3 erlaubt einen Vortrieb des Mittels zur Aufnahme von Kettenbestandteilen 2 konzentrisch zu ihrem Mittelpunkt. Somit ist keine äußere Antriebskraft des Magazins zum Auswurf eines Kettenbestandteils nötig. Das Magazin ist voll sterilisierbar. Es bedarf zum Auswurf eines Kettenbestandteils lediglich eines Impulses von außen an den Auswerferhebel, damit der Vortrieb im Inneren selbständig vorgenommen werden kann. Als Lagerfläche für das Mittel zur Aufnahme von Kettenbestandteilen 2 dient eine Materialwand des Gehäuses 7 als innerer Lagerring 8. Der innere Lagerring 8 ist bevorzugt um die Öffnung 17 herum ausgebildet. Ferner ist auf dem Mittel zur Aufnahme von Kettenbestandteilen 2 ein Zahnkranz 13, bevorzugt mit einem kleineren Durchmesser als das Mittel zur Aufnahme von Kettenbestandteilen 2, angeordnet. Der Zahnkranz 13 steht dabei bevorzugt in Wirkverbindung zum Mittel zur Aufnahme von Kettenbestandteilen 2 oder direkt zur Konstantkraftfeder 3 und ist ebenfalls drehbar im Gehäuse 7 gelagert. Der Zahnkranz 13 ist fest mit dem Mittel zur Aufnahme von Kettenbestandteilen 2 gegen Verdrehung zueinander verbunden. Er dient der Begrenzung des Vortriebs der Konstantkraftfeder 3 während des Auswurfs, so dass immer nur ein Kettenbestandteil ausgeworfen werden kann. Bevorzugt sind Zahnkranz 13 und Mittel zur Aufnahme von Kettenbestandteilen einstückig ausgebildet.

Vom Zentrum des Magazins 1 nach außen hin besteht das Magazin 1 aus einer zentralen Öffnung 17, einem inneren Lagerring 8, dem Zahnkranz 13, das Mittel zur Aufnahme von Kettenbestandteilen 2 und einer Begrenzung 16 des Mittels zur Aufnahme von Kettenbestandteilen 2. Im vorliegenden Ausführungsbeispiel sind Zahnkranz 13 und das Mittel zur Aufnahme von Kettenbestandteilen 2 in einer Vertiefung in die hintere Gehäuseschale 7b eingelassen.

Die innere Wandung 16 der Vertiefung (s. Fig. 3) dient dabei der Begrenzung des Mittels zur Aufnahme von Kettenbestandteilen 2. Der Abstand zwischen Wandung 16 der Vertiefung oder allgemeiner, der Begrenzung 16 des Mittels zur Aufnahme von Kettenbestandteilen 2 und des Mittels zur Aufnahme von Kettenbestandteilen 2 selbst ist so dimensioniert, dass die Kettenbestandteile in den Vertiefungen 6 ohne herauszufallen führbar gelagert werden können. Die Kettenbestandteile werden dabei auf einer Kreisbahn zum Auswurf 10 geführt. Unter dem Mittel zur Aufnahme von Kettenbestandteilen 2 ist die Spannfeder 3 gelagert, wie in Fig. 3 gezeigt.

Die Feder 3 und damit das Mittel zur Aufnahme von Kettenbestandteilen 2 werden durch den Auswerferhebel 4 wie weiter unten beschrieben gehemmt. Die Hemmung der Feder 3 und des Mittels zur Aufnahme von Kettenbestandteilen 2 gestattet immer nur das Freisetzen eines Implantats bzw. Kettenbestandteils aus dem Magazin pro Betätigung des Magazins. Die Hemmung erfolgt zum einen durch den Formschluss des ersten Implantats an dem Auswerferhebel 4 und zum anderen durch die Taktverzahnung des oberen Zahnkranzes 13. Der Auswerferhebel 4 unterstützt außerdem das Freisetzen des Implantats, indem er es aktiv in einen Arbeitskanal schiebt. Der Auswerferhebel 4 ist bevorzugt federngelagert und wird durch einen entsprechenden Hebelmechanismus im später beschriebenen Gerät zum Beladen 101 betätigt. Die Implantate werden aus der Öffnung 10 an der Magazinunterseite in einen Arbeitskanal freigesetzt. Bevorzugt sind die Magazine 1 mechanisch codiert und farblich markiert. Die farbliche Markierung kann durch Färbung des Mittels zur Aufnahme von Kettenbestandteilen 2 erfolgen, die den beweglichen Teil der Skala darstellt. Die Anzahl der verbrauchten Strahlenquellen und Abstandshalter können direkt an den Magazinen abgelesen werden.

Im Folgenden wird die Beschreibung des Magazins beispielhaft anhand eines Strahlenquellenmagazins beschrieben. Dies erfolgt jedoch nicht beschränkend. Die Ausführungen gelten auch für Abstandshaltermagazine, es sei denn etwas Gegenteiliges ist explizit erwähnt.

Zur Montage des Magazins 1 wird zuerst die Spannfeder 3 and die hintere Gehäusehälfte montiert und dann gemeinsam mit dem Mittel zur Aufnahme von Kettenbestandteilen 2 in die hintere Gehäuseschale 7b eingesetzt. Anschließend werden ein Auswerferhebel 4 und ein Verschlussschieber 5 eingesetzt und jeweils verspannt, bevorzugt mit Druckfedern 20 und 21, wie in Fig. 3 gezeigt. Hierbei ist der Auswerferhebel 4 über einen Stift rotatorisch gelagert und der Verschlussschieber 5 kann in einer Nut in der Gehäuseschale 7b translatorisch verschoben werden.

Nach der Montage des Magazins 1 ohne Deckel 7a werden in den halbkreisförmigen Vertiefungen 6 an der Stirnseite des Mittels zur Aufnahme von Kettenbestandteilen 2 die Strahlenquellen 19 (oder Abstandshalter 18) parallel zur Achse des Mittels zur Aufnahme von Kettenbestandteilen 2 positioniert. Hierbei muss vor dem Beladen des Mittels zur Aufnahme von Kettenbestandteilen 2 die Spannfeder 3, bevorzugt eine Konstantkraftfeder 3 gespannt werden. Dies erfolgt durch Verdrehen des Mittels zur Aufnahme von Kettenbestandteilen 2 in Spannrichtung der Spannfeder 3. Ein Blockieren gegen das Entspannen der Spannfeder 3 erfolgt durch das Setzen der ersten Strahlenquellen 19. Die weiteren Strahlenquellen 19 werden anschließend eingesetzt. Im späteren Betrieb des Magazins 1 dient die jeweilig erste Strahlenquellen 19 vor ihrer Freigabe immer als Blockierelement oder Mittel zum Blockieren gegen das Entspannen der Spannfeder 3. Nach dem Befüllen wird das Magazin 1 mit einem Deckel 7a versehen. Ein innerer Lagerring 8 des Mittels zur Aufnahme von Kettenbestandteilen 2 ist verbreitert und dient mit einer Markierung 15 versehen gemeinsam mit dem Magazindeckel 7a als Inhaltsanzeige für die noch in den Magazinen 1 verbliebenen Strahlenquellen 19 bzw. Abstandshalter 18.

Das Magazin 1 für die Lagerung von Abstandshaltern 18 ist mit Ausnahme des Verschlussschiebers 5 und der entsprechenden Ausfräsung in der hinteren Gehäuseschale 7b baugleich mit dem Strahlenquellenmagazin. Daher erfolgen die Montage und das Befüllen beider Magazintypen analog. Der Verschlussschieber 5 kann im Abstandshaltermagazin ebenfalls angeordnet sein. Er ist aber nicht zwingend notwendig, da die Abstandshalter nicht radioaktiv sind. Der Verschlussschieber 5 des Strahlenquellenmagazins dient der Abschirmung der Strahlung nach Außen, bevor das Magazin in ein Gerät 101 zum Befüllen eingeführt wird.

Mindestens eine, bevorzugt beide schmalen Seitenflächen eines Magazins 1 sind mit Sicherungsnuten 9 versehen. Diese dienen nach dem Einsetzen der Magazine 1 in ein Gerät 101 zur Konfektionierung von Strahlenquellenkette als Sicherung gegen ein Herausfallen aus dem Gerät sowie als Positionierhilfe gegenüber einem Arbeitskanal im Gerät 101.

Im Mittenbereich der Unterfläche des Strahlenquellenmagazins befindet sich eine Öffnung zum Auswurf der Implantate 10. Auf der einen Seite dieser Öffnung 10 ist die Seitenfläche mit einer Kodierungsbohrung 11 versehen. Beim Einsetzen des Strahlenquellenmagazins in ein Gerät zum Fügen von Strahlenquellenketten 101 wird durch einen Kodierungsdorn im Gerät 101 der Verschlussschieber 5 so verschoben, dass er die Auswurföffnung 10 freigibt. Diese Funktion existiert bevorzugt im Abstandshaltermagazin nicht, da der Verschlussschieber 5 verhindern soll, einen Operateur radioaktiver Strahlung aus den Strahlenquellen 19 auszusetzen. Dies ist bei Abstandshaltern 18 nicht notwendig, da sie nicht radioaktiv sind. Natürlich kann aber dennoch der Schieber 5 vorgesehen sein.

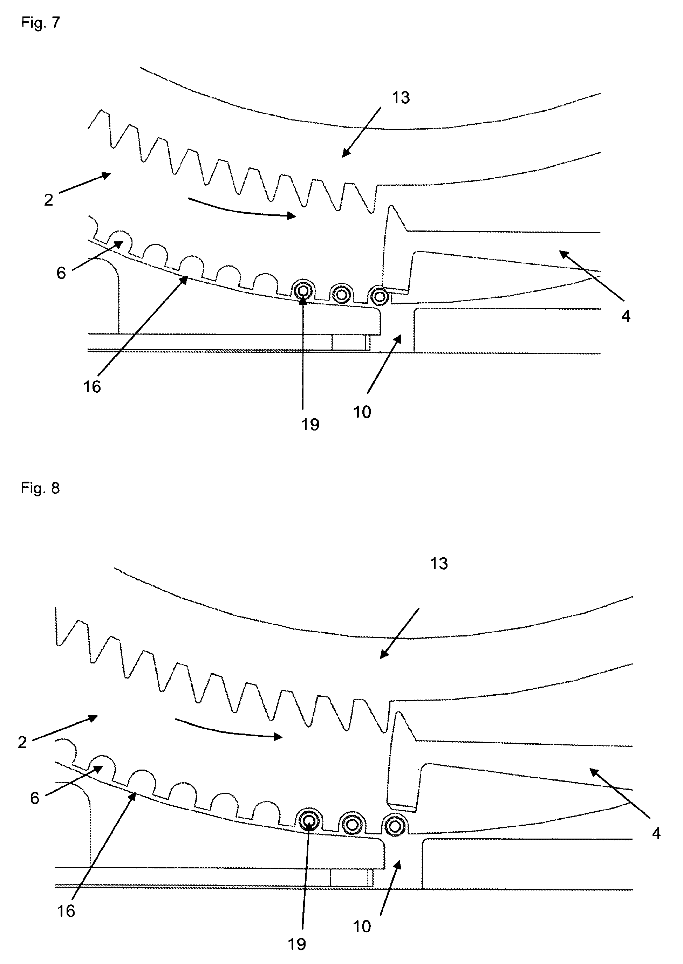

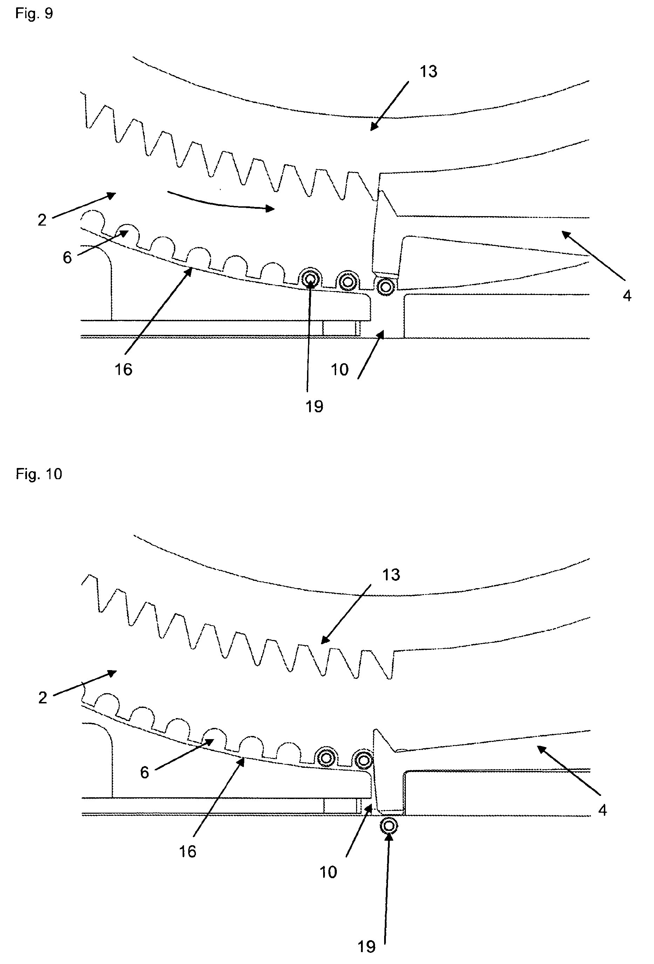

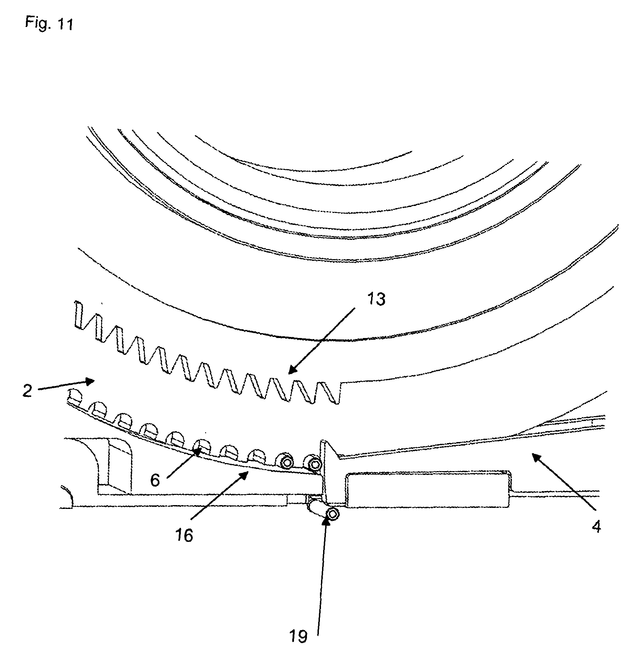

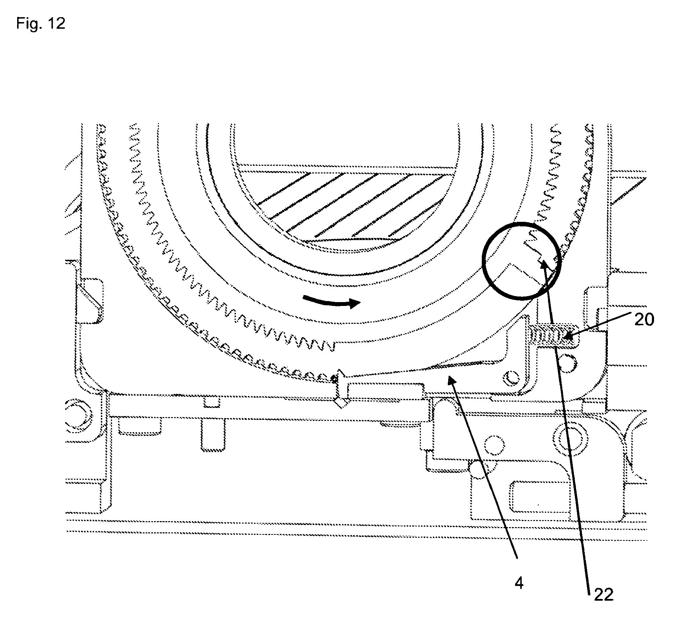

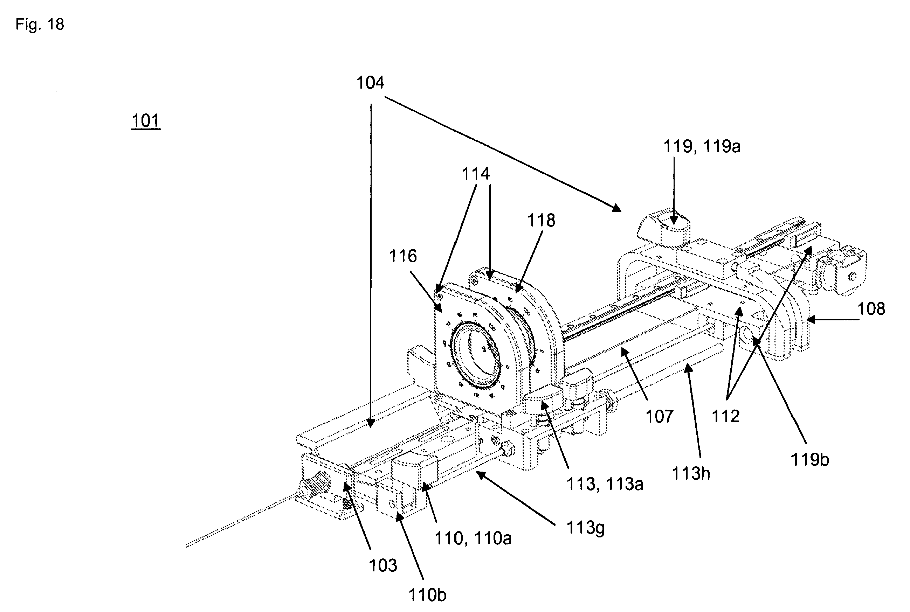

Durch eine zweite Öffnung 12 auf der anderen Seite der Auswurföffnung 10 lässt sich der Auswurfhebel 4 durch einen entsprechenden Hebelmechanismus 113 im Gerät 101 betätigen. Der Auswurfmechanismus ist in den Figuren 6 bis 11 in einzelnen Teilschritten dargestellt. Zunächst ist wie in Fig. 6 dargestellt der Auswurf 10 durch den Auswurfhebel 4 physisch blockiert. Der Auswurfhebel 4 greift in dieser Position nicht in den Zahnkranz 13 ein. Das Auswerfen jeweils einer Strahlenquelle 19 bzw. eines Abstandshalters 18 aus den Magazinen 1 erfolgt durch ein Betätigen der äußeren Fläche des Auswurfhebels 4. Dadurch gleitet der Auswurfhebel 4 an der ersten Strahlenquelle 19 bzw. dem ersten Abstandshalter 18 nach oben hin ab (Fig. 7). Die erste Strahlenquelle 19 bzw. Abstandshalter 18 verliert seine Blockierfunktion der Fig. 6, das Mittel zur Aufnahme von Kettenbestandteilen 2 bewegt sich getrieben durch die gespannte Feder 3 konzentrisch zur Lochöffnung 17 oder Mitte des Magazins 1. Diese Bewegung wird jedoch durch das Eingreifen des Auswurfhebels 4 in die Taktverzahnung des oberen Zahnkranzes 13 des Mittels zur Aufnahme von Kettenbestandteilen 2 im Rahmen der weiteren Bewegung blockiert (Fig. 8, 9). Durch diese Drehung des Mittels zur Aufnahme von Kettenbestandteilen 2 wird die erste Strahlenquelle (bzw. Abstandshalter) und nur diese in eine Position befördert, die über der Auswurföffnung 10 liegt (Fig. 9). Die erste Strahlenquelle wird dann durch den Auswerferhebel 4 nach unten hinten, bevorzugt in einen Arbeitskanal (nicht gezeigt) unter dem Auswurf 10 zum Zusammenfügen der Kettenbestandteile freigesetzt (Fig. 10 und 11). Nach dem Betätigen des Auswurfhebels 4 fällt dieser wieder in seine vorherige Position zurück und blockiert wiederum den Auswurf 10 wie in Fig. 6. Somit wird die weitere Drehung des Mittels zur Aufnahme von Kettenbestandteilen 2 erneut durch das Anliegen der nächsten ersten Strahlenquelle 19 bzw. des nächsten ersten Abstandshalters 18 am Auswurfhebel 4 verhindert (Fig. 11). Durch diesen Mechanismus wird eine schrittweise Drehung des Mittels zur Aufnahme von Kettenbestandteilen 2 und ein Einzelabwurf der Strahlenquellen 19 bzw. Abstandshalter 18 in einen Arbeitskanal realisiert. Ein Auslösen der Hebel 4 zur Magazinbetätigung bei leeren Magazinen 1 ist nicht möglich. Dies ist in Fig. 12 dargestellt. Mit einem Pfeil ist die Drehrichtung des Mittels zur Aufnahme von Kettenbestandteilen 2 dargestellt. Das letzte Implantat im Magazin ist an der mit einem Kreis markierten Stelle angeordnet. Nach dem Auswurf des letzten Implantats aus dem Magazin, fällt der Hebel 4 gezwungen durch die Antriebsfeder 3 und die Hebelfeder, die erste Druckfeder 20, in die eingekreiste Einkerbung des Blockierelementes 22. Das Blockierelement 22 bietet keinen Platz für eine Auslenkung des Hebels 4. Das Betätigen der Tasten 113a des Hebelmechanismus 113 zum Auswerfen von Kettenbestandteilen (s. Fig. 18) bewirkt über einen Hebelmechanismus 113 ein Auslenken der Hebel 4. Da dieses Auslenken jedoch blockiert ist, ist auch das Betätigen der Tasten 113a bei leerem Magazin blockiert.

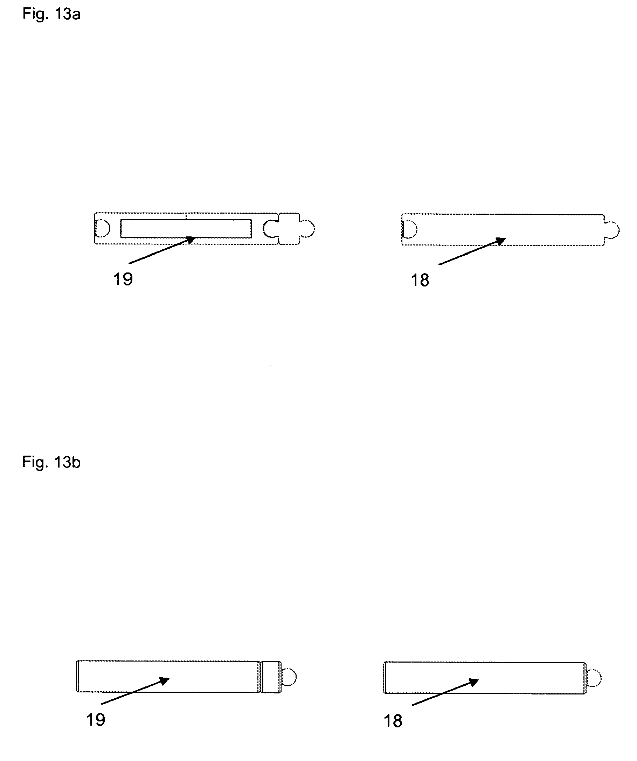

Fig. 13 zeigt zusammenfügbare Abstandshalter 18 und Strahlenquellen 19. Fig. 13a zeigt die Abstandshalter 18 und Strahlenquellen 19 im Querschnitt entlang einer Längsachse und Fig. 13b zeigt Abstandshalter 18 und Strahlenquelle 19 von außen betrachtet. Die Strahlenquellen 19 weisen dabei einen inneren radioaktiven Kern auf, in Fig. 13a als Rechteck dargestellt. Die Strahlenquellen 19 und Abstandshalter 18 weisen Enden auf, die so ausgebildet sind, dass sie zu einer Kette zusammengefügt werden können. Es können dabei auch zwei Abstandshalter 18 oder zwei Strahlenquellen 19 direkt zusammengefügt werden. Die Abstandshalter 18 bzw. Strahlenquellen 19 weisen bevorzugt jeweils männliche Enden (rechts) und weibliche Enden (links) auf. Durch die Ausbildung der Strahlenquellen 19 und Abstandshalter 18 sind die Ketten frei konfektionierbar.

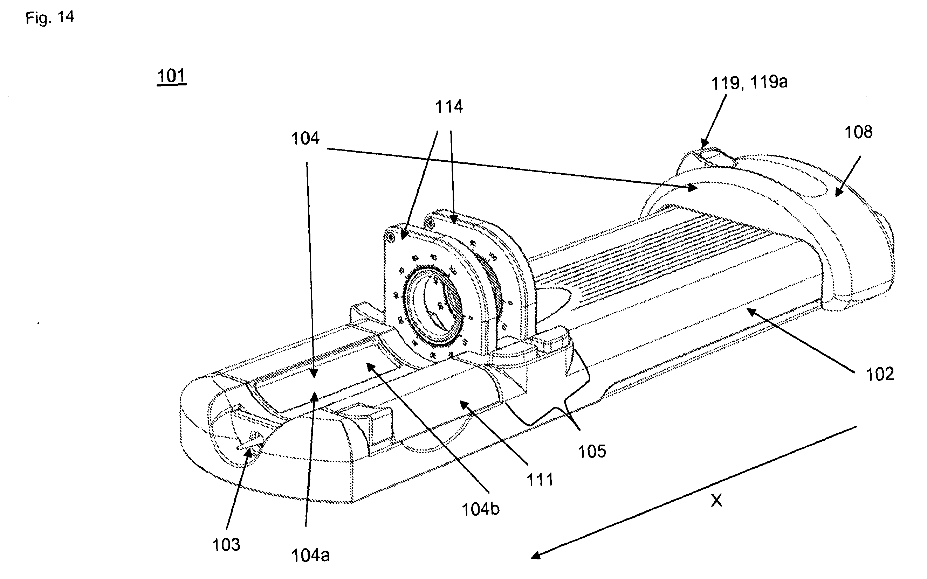

Fig. 14 zeigt nun eine perspektivische Ansicht eines erfindungsgemäßen Gerätes 101 zum Fügen und Konfektionieren von Ketten, die radioaktive Strahlenquellen beinhalten, welches bevorzugt mit den oben beschriebenen Magazinen 1 betrieben wird.

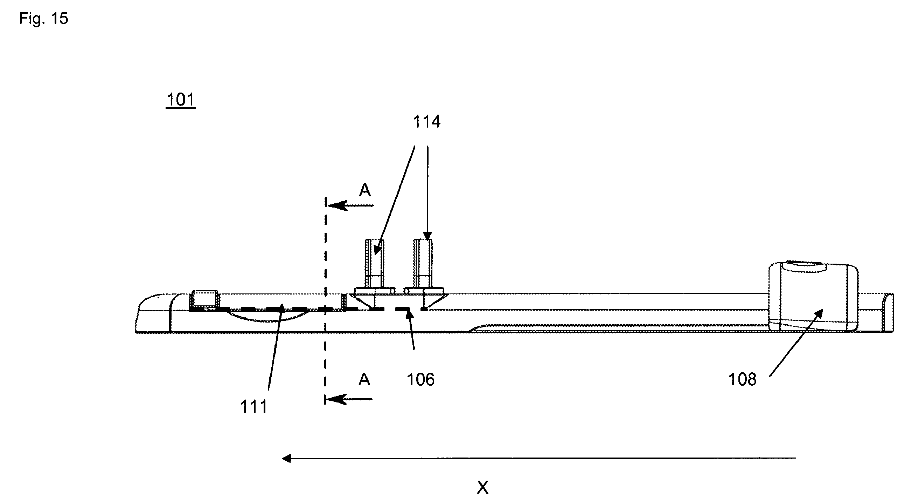

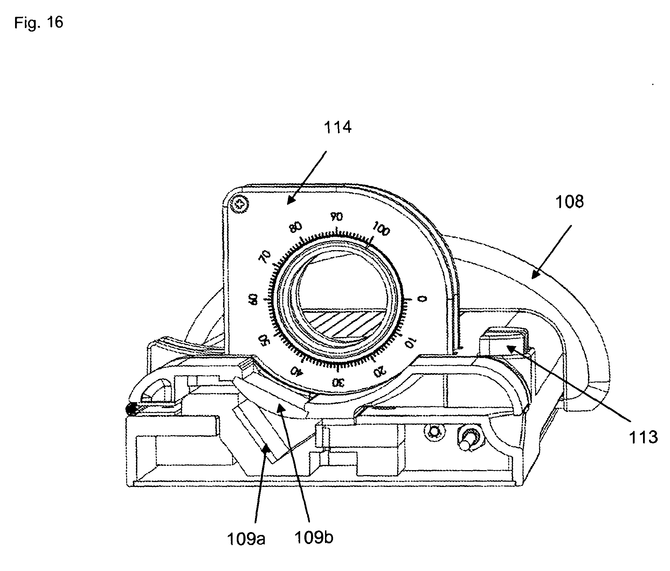

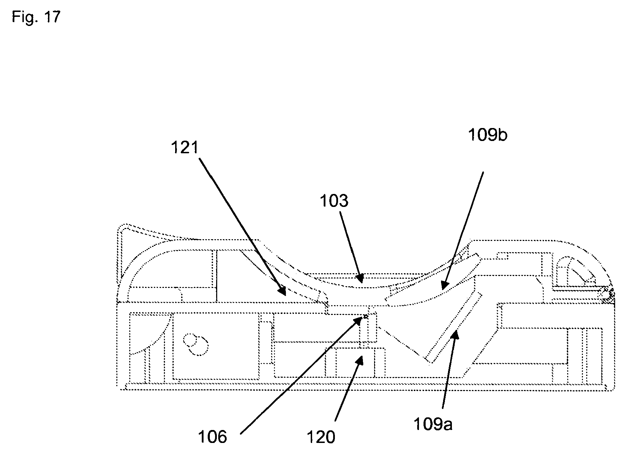

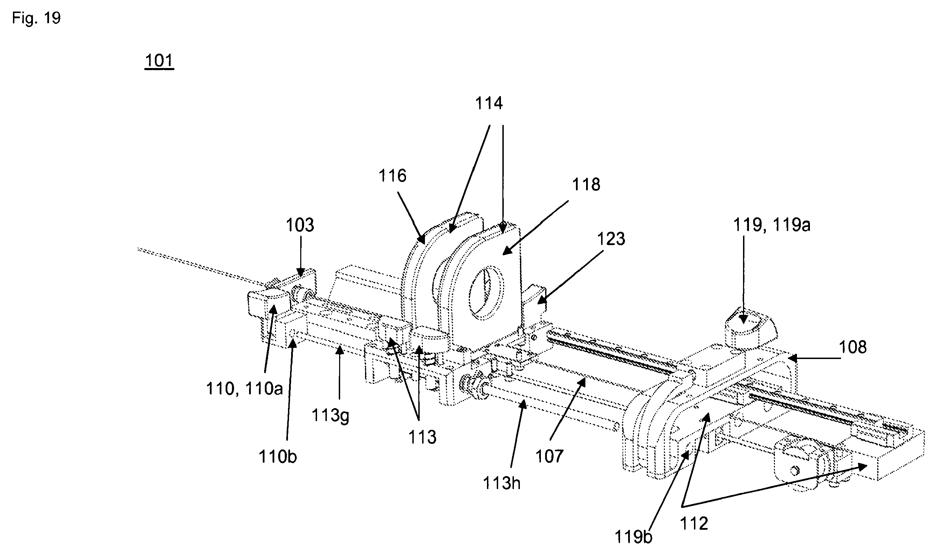

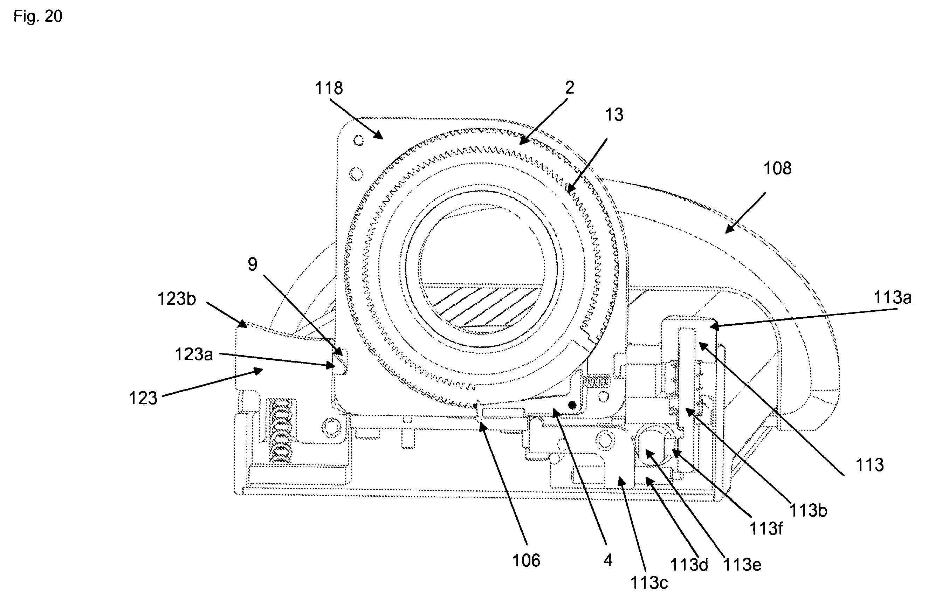

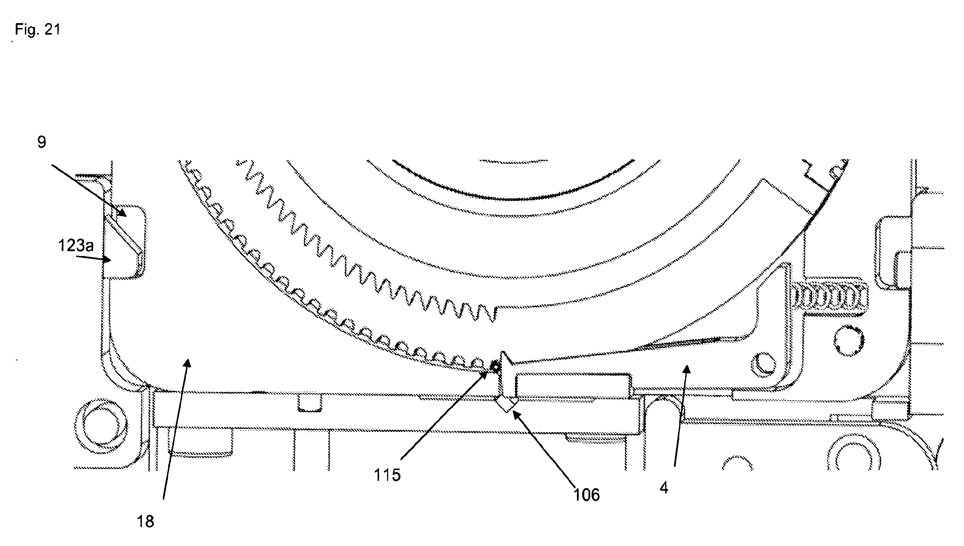

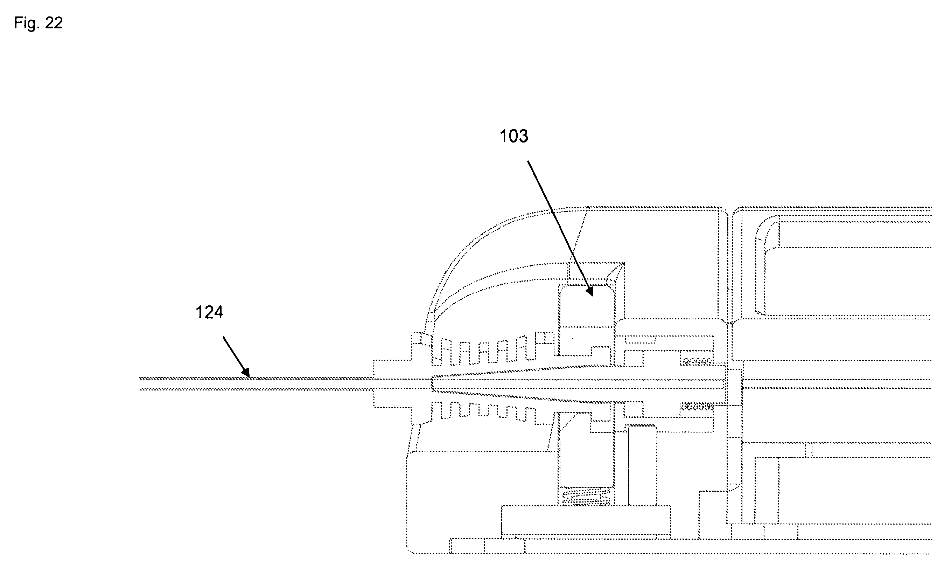

Fig. 15 zeigt eine Seitenansicht des erfindungsgemäßen Gerätes zum Fügen und Konfektionieren von Ketten der Fig. 14. Fig. 16 ist ein perspektivischer Querschnitt durch das erfindungsgemäße Gerät entlang der Achse A - A in Fig. 15, gesehen entgegen der Richtung der Pfeile der Fig. 15. Fig. 17 zeigt einen Querschnitt durch das erfindungsgemäße Gerät entlang der Achse A-A in Fig. 15, gesehen in Richtung der Pfeile der Fig. 15. Fig. 18 ist eine erste Ansicht des erfindungsgemäßen Gerätes ohne Gehäuse. Fig. 19 ist eine zweite Ansicht des erfindungsgemäßen Gerätes ohne Gehäuse. Fig. 20 zeigt einen Querschnitt durch das Gerät zum Fügen durch ein Abstandshaltermagazin. Fig. 21 stellt eine Vergrößerung der Fig. 20 im unteren Bereich des Abstandshaltermagazins dar. Fig. 22 zeigt einen Längsschnitt durch den vorderen Bereich des erfindungsgemäßen Gerätes zum Fügen im Bereich des Nadelhalters.

Das erfindungsgemäß beschriebene Gerät 101 besteht wie in Fig. 14 gezeigt aus einem Gehäuse 102, einem Nadelhalter 103, einer Füge- und Sichteinheit 104 sowie einer Beladungseinheit 105. In die Beladungseinheit werden Magazine 114 für Kettenbestandteile eingesetzt. Die Füge- und Sichteinheit 104 umfasst bevorzugt einen Fügebereich 104a in dem die Kettenelemente zusammengefügt werden sowie eine Sichtbereich 104b zur Überprüfung der Anordnung der Kettenbestandteile. In einem bevorzugten Ausführungsbeispiel ist der Fügebereich 104a über eine Klappe 111 von außen erreichbar. An einem Ende des Gerätes ist der Nadelhalter 103 angeordnet, an dem anderen Ende ein Griff 108, der noch weiter erläutert werden wird. Am Griff 108 ist eine dritte Sperre 119, ausgebildet mit einem Tastelement 119a angeordnet. Zwischen Griff 108 und Nadelhalter 103 sind die Magazine 114 und der Füge- bzw. Sichtbereich 104a, 104b angeordnet.

Die Aufgabe des Gerätes 101 besteht darin, mit seiner Hilfe radioaktive Strahlenquellen 115 und inaktive Abstandshalter 117 aus entsprechenden Magazinen 114 freizusetzen, diese mit Steckverbindungen versehenden Implantate (s. Fig. 13) oder Kettenbestandteile 115, 117 zu einer Kette zusammenzufügen, um mit diesen anschließend die dem Gerät 101 aufgesteckten lmplantationsnadeln 124 zu befüllen (s. Fig. 22).

Zentrales Element des beschriebenen Gerätes 101 stellt der Arbeitskanal 106 dar, der sich wie in Fig. 15, 17, 19 und 20 gezeigt entlang der Achse X bzw. entlang einer Längesachse des Gerätes 101 ausdehnt. Der Arbeitskanal 106 beginnt vom Griff 108 aus gesehen bevorzugt kurz vor den Magazinhalterungen oder alternativ genau unterhalb des Auswurfs des dem Griff nächsten Magazins 114 und endet am Nadelhalter 113. Ein Beginn des Arbeitskanals 106 kurz vor den Magazinhalterungen ermöglicht eine bessere Führung des später zu beschreibenden Mandrels 107. Um den Arbeitskanal 106 herum sind die verschiedenen Einheiten angeordnet, gehalten durch das Gehäuse 102. Das Gehäuse 102 trägt und schützt die sonstigen Funktionseinheiten des Gerätes 101. Von rechts nach links ist in Fig. 14 und Fig. 15 zunächst der Griff 108, die Magazine 114 mit der Beladungseinheit 105, der Füge- und Sichtbereich 104a, 104b und der Nadelhalter 103 angeordnet.

Die Füge- und Sichteinheit 104 umfasst den Arbeitskanal 106 und beinhaltet einen Mandrel 107 (s. Fig. 18 oder 19), der über einen äußeren Griff 108 im Arbeitskanal 106 linear geführt ist sowie eine Spiegel-Linsen-Einheit 109 (s. Fig. 17) zur Visualisierung der Implantate im Fügebereich 104a. Der Mandrel 107 transportiert über den Griff 108, der durch den Anwender lateral verschoben werden kann, die aus dem Magazin freigegebenen Strahlenquellen 115 bzw. Abstandshalter 117 aus dem Teil des Arbeitskanales 106 unter den Magazinen 114 in den Fügebereich 104a. Im Fügebereich 104a kann die Konfiguration vorteilhafterweise über die Sichteinheit 104b überprüft werden. Nachdem die gewünschte Konfiguration der Implantate erstellt ist, werden diese im Fügebereich 104a mit Hilfe des Mandrel 107 zusammengeschoben und so zu einer Seed-Spacer-Kette gefügt. Als Gegenlager blockiert eine erste Sperre 110, die einen Taster 110a umfasst, während des Fügens den Übergang von Arbeitskanal 106 zum Nadelhalter 103. Nach dem Fügen wird die erste Sperre 110 geöffnet und erlaubt so das Ausschieben der gefügten Implantatkette über den Nadelhalter 103 in die Nadel 124 (s. Fig. 22). Die erste Sperre 110 ist hierbei bevorzugt vor dem Nadelhalter 103 und hinter dem Sichtbereich 104b angeordnet, kann aber auch mit dem Nadelhalter 103 zusammenfallen.

Der manuell bedienbare Nadelhalter 103 besteht aus einer Verschlusseinrichtung und einem Nadeladapter, der die gefügte lmplantatkette in die aufgesteckte Nadel 124 leitet. Die Verschlusseinrichtung des Nadelhalters hält und sichert die Nadel 124 gegen Verlust beim Befüllen.

Ein Linsen-Spiegel-System 109 erlaubt einen indirekten und somit strahlungsgeschützten Sichtkontakt zu den Implantaten bzw. Kettenbestandteilen. Über eine Klappe 111 in der Sichteinheit 104b kann bei Bedarf direkt in den Arbeitskanal 106 eingegriffen werden, um so eventuelle Korrekturen an der Seed-Spacer-Konfiguration vorzunehmen. Für eine kurzzeitige Zwischenlagerung der Strahlenquellen bzw. Abstandshalter während einer Korrektur stehen unterhalb der strahlengeschützten Klappe 111 zwei muldenförmigen Lagerplätze zur Verfügung.

Beide Magazine 114 sind während des Betriebes im Gerät 101 über einen Sicherungsmechanismus 123 wie in Fig. 20 gezeigt einrastend fixiert und gegen ein Herausfallen gesichert. Der Sicherungsmechanismus 123 umfasst hierbei einen Riegel, der in eine Sicherungsnut 9 eines Abstandshaltermagazins 18 eingreift. Die Magazine 114 können jedoch jederzeit durch Betätigen eines entsprechenden Auslösehebels entriegelt und entnommen werden. Ein Vertauschen der Magazine 114 in ihren bestimmungsgemäßen Steckplätzen ist durch eine mechanische Kodierung nicht möglich. So umfasst bevorzugt das Strahlenquellenmagazin 116 eine Kodierung, die von der Kodierung des Abstandshaltermagazins 118 verschieden ist. Die Kodierung kann durch einen Kodierungsdorn erfolgen, der in eine entsprechende Kodierungsöffnung 11 des Magazins eingreift. Die Kodierung des Strahlenquellenmagazins 116 verschiebt beim Einsetzen des Magazins 116 noch zusätzlich einen o.g. Verschlussschieber 5. Dieser schützt zusätzlich vor einer Strahlungsexposition beim Umgang und während des Transports des Magazins 116 vor dem Einsetzen in das Gerät 101. Nach Entnahme des Strahlenquellenmagazins 116 schließt auch wieder der Verschlussschieber 5.

Um Beschädigungen an den Implantaten durch zu große Kräfte beim Fügen der Implantate zu vermeiden, ist die Fügeeinheit 104 mit einer Magnetkupplung 122 versehen. Hierzu ist der den Griff 108 und den Mandrel 107 führende Schlitten 112 zweigeteilt ausgebildet, wie in Fig. 18 und Fig. 19 gezeigt. Beide Teile trennen sich bei Überbelastung der Haftkraft der Magnetkraft voneinander.

Die Beladungseinheit 105 beinhaltet die Steckplätze für ein Abstandshalter- und ein Strahlenquellenmagazin 118, 116 sowie einen mechanischen Hebelmechanismus 113 zum Freisetzen der Implantate. Nach Einsetzen eines Magazins 114 und Betätigung einer entsprechenden Taste 113a im Hebelmechanismus 113 wird ein Implantat aus dem Magazin in den darunterliegenden Kanal 106 freigesetzt. Der Hebelmechanismus 113 pro Magazin 114 besteht aus einem Taster 113a, der bevorzugt gleitgelagert ist, einer Tasterstange 113b mit einer Quernut, wobei die Tasterstange 113b bevorzugt federnd gelagert und einseitig, zum ersten Federdruckstück 113d hin abgeflacht ist. Ferner umfasst der Hebelmechanismus 113 einem im Gehäuse rotatorisch und federnd gelagerten Hebel 113c mit einem erstem Federdruckstück 113d und einer quer zu den Tasterstangen 113b translatorisch geführten Schubstange 113e mit jeweils einem zweitem Federdruckstück 113f für jeden der Hebelmechanismen sowie eine erste Verlängerung 113g der Schubstange und eine zweite Verlängerung 113h der Schubstange.

Der Hebelmechanismus 113 wird im Folgenden unter Bezug auf die Figuren 18-20 näher erläutert werden. Durch Drücken auf den Taster 113a wird die bevorzugt beidseitig gleitlagergeführte Tasterstange 113b nach unten bewegt (s. Fig. 20). Hier drückt sie mit ihrem freien Ende auf den federnd gelagerten Zapfen des ersten Federdruckstückes 113d, wodurch das Federdruckstück 113d nach unten gedrückt wird. Da dieses Federdruckstück 113d direkt mit dem im Gehäuse drehbar gelagerten Hebel 113c verbunden ist, überträgt sie diese Bewegung direkt auf den Hebel 113c. Der Hebel 113c hebt dadurch sein dem Federdruckstück 113d gegenüberliegendes Ende, welches in das darüber angeordnete Magazin eingreift und hierdurch die Freigabe eines Implantates auslöst.

Um den Taster 113a nach dem Betätigen in der untere Position zu fixieren, rastet ein zweites Federdruckstück 113f der Schubstange 113e in eine Quernut der abgeflachten Tasterstange 113b ein. Gleichzeitig gleitet der federnde Zapfen des ersten Federdruckstückes 113d des Hebels 113c an der abgeflachten Tasterstange 113b ab. Da die Hebel 113c federnd gelagert sind, stellen sie sich dadurch in ihre Ursprungslage zurück. Zweck dieses Mechanismus ist es, aus Sicherheitsgründen den Taster 113a nach Betätigung temporär in der gedrückten Position zu fixieren, jedoch den Hebel 113c direkt nach Auslösen des Magazins 114 in seine Ursprungslage zurückkehren zu lassen.

Wie in Fig. 18 und 19 zu sehen, ist die Schubstange 113e zu beiden Seiten des Hebelmechanismus 113 verlängert, mit einer ersten Verlängerung 113g und einer zweiten Verlängerung 113h. Die erste Verlängerung 113g der Schubstange 113b ist zwischen den Magazinen 114 und der ersten Sperre 110 angeordnet und kann zur Öffnung der ersten Sperre 110 in eine Öffnung 110b der ersten Sperre 110 eingreifen und den Taster 110a der ersten Sperre 110 öffnen. Die zweite Verlängerung 113h der Schubstange 113b ist zwischen den Magazinen und dem Griff 108 angeordnet und greift in eine Öffnung 119b der dritten Sperre 119 ein.

Wurde unter den Magazinen 114 Implantate 115, 117 freigesetzt und die Taster 113a verbleiben in der unteren Stellung, muss erst der Griff 109 in X-Richtung verschoben werden. Wird der Griff in X-Richtung verschoben und ist die dritte Sperre 119 geschlossen, schiebt der Nutzer mit dem Griff 108 die dritte Sperre 119 gegen die eine zweite Verlängerung 113h und somit gegen die Schubstange 113e. In diesem Zustand ist die zweite Verlängerung 107 in der Öffnung 119b der dritten Sperre 119 fixiert und wird mit dem Griff 108 verschoben. Die zweiten Federdruckstücke 113f der Schubstange 113e werden dann aus den Quernuten der Tasterstange 113b geschoben und somit die Taster 113a entriegelt. Da mit der Bewegung des Griffes 108 auch der Mandrel 107 bewegt wird, wurden gleichzeitig mit dem Entriegeln der Taster 113a die Implantate aus dem Raum unter den Magazinen verschoben.

Ist die dritte Sperre 119 geöffnet, kann der Griff 108 und der Mandrel 107 in X-Richtung verschoben werden, ohne von der dritten Sperre 119 und der zweiten Schubstangenverlängerung 113h begrenzt zu werden. Diese Stellung wird verwendet, um die Implantatketten zu fügen und später in die Nadel 123 zu schieben. In dieser Position der dritten Sperre 119 können jedoch die Taster 113a nicht wieder entriegelt werden.

Wurde die erste Sperre 110 geöffnet, um die Implantatkette nach dem Fügen in die Nadel 123 zu schieben, wird sie in dieser Position fixiert, um Beschädigungen der Kette durch die zurückfallende Sperre 110 zu vermeiden. Sie wird erst durch einen Impuls der ersten Verlängerung 113g der Schubstange 113e über die Öffnung 110b wieder verschlossen, wenn der Schlitten mit der dritten Sperre 119 die zweite Verlängerung 113h verschiebt. Im geschlossenen Zustand dient die erste Sperre 110 als Gegenlager zum Fügen der Implantatketten aus den Einzelimplantaten.

Durch den Hebelmechanismus 113 kann somit ein Auswerfen von zwei Kettenbestandteilen übereinander vermieden werden. Allerdings können die Taster 113a unterschiedlicher Magazine 114 auch gleichzeitig ausgelöst werden, da sie beabstandet über dem Arbeitskanal 106 angeordnet sind. Vorteilhafterweise kann somit das Konfektionieren durch gleichzeitiges Drücken (oder kurz hintereinander) von Strahlenquellen- und Abstandshaltermagazintasten 113a beschleunigt werden.

Fig. 20 zeigt einen Querschnitt durch das erfindungsgemäße Gerät 101 in Höhe des Abstandshaltermagazins 118 durch dieses hindurch. Hinter dem Abstandshaltermagazin 118 ist der Griff 108 sichtbar. Auf Höhe des Abstandshaltermagazins 118 befindet sich der Sicherungsmechanismus 123 der Aufnahmeeinrichtung für Magazine des Geräts 101. Der Sicherungsmechanismus 123 umfasst einen Vorsprung 123a, in Fig. 20 exemplarisch an seiner rechten oberen Seite angeordnet, der in die Sicherungsnut 9 des Abstandshaltermagazins 118 eingreift und das Magazin 118 im Gerät 101 gegen ein Herausfallen sichert. Der Sicherungsmechanismus 123 umfasst ferner eine Taste 123b im oberen Bereich. Durch Drücken der Taste 123b wird der Vorsprung 123a des Sicherungsmechanismus aus der Sicherungsnut 9 herausgeschwenkt und gibt das Magazin 118 frei. In Fig. 20 ist der Sicherungsmechanismus 123 auf einer Seite des Abstandshaltermagazins 118 angeordnet, der Auswerf-Hebelmechanismus 113 auf der anderen Seite. Der Auswerf-Hebelmechanismus 113 umfasst ebenfalls eine Taste 113a, die in Wirkverbindung mit dem Auswerferhebel 4 des Magazins 118 steht. Durch Drücken der Taste 113a des Hebelmechanismus 113 wird der Auswerferhebel 4 nach oben rotiert und ein Abstandshalter 119 nach unten in den Arbeitskanal 106 freigegeben. Fig. 21 zeigt die Anordnung des Arbeitskanals 106 der Fig. 20 in einer Vergrößerung. Der Arbeitskanal 106 hat bevorzugt eine V-Form, so dass die Kettenbestandteile im tiefsten Punkt des V's gelagert und geführt werden.

Fig. 22 zeigt eine Nadel 124, die in den Nadelhalter 103 des Gerätes 101 eingesteckt ist. Die Kettenbestandteile werden als gefügte Kette aus der Füge- und Sichteinheit in die Nadel 124 mittels des Mandrels 107 geschoben.

Es soll im Folgenden noch einmal in kurzen Worten die Reihenfolge der Betätigung der Elemente des Gerätes 101 zum Zusammenfügen von Kettenbestandteilen erläutert werden:
- Betätigen des Hebelmechanismus 113 und Auswerfen eines Kettenbestandteils; Blockierung des Hebelmechanismus 113 des betätigten Magazins;
- Verschiebung des Griffs 108 zur Bewegung des Mandrels 107 entlang der Gerätelängsachse im Arbeitskanal 106 zur Verschiebung des ausgeworfenen Kettenbestandteils in den Füge- und Sichtbereich 104a, 104b; Freigabe des zuvor blockierten Hebelmechanismus 113;
- Wiederholung von Schritt 1 und 2 bis die benötigten Kettenbestandteile freigegeben sind;
- Drücken und Einrasten der Taste 119a der der dritten Sperre 119 am Griff 108 zum Zusammenfügen der Kettenbestandteile durch Zusammendrücken der Kettenbestandteile mittels des Mandrels 107 gegen die erste Sperre 110;
- Freigabe der ersten Sperre 110 durch Drücken der Taste 110a der ersten Sperre 110;
- Verschieben des Mandrels 107 zum Verschieben der zusammengefügten Kettenbestandteile in eine Nadel 124.
- federngetriebenes oder manuelles Zurückfahren des Griffes 108 in seine Ursprungslage; erst nach erneutem Verschieben des Mandrel 107 wird die erste Sperre 110 wieder geschlossen, bevorzugt über den Eingriff der ersten Verlängerung 113g in die erste Öffnung 110b der ersten Sperre 110.

Zusammenfassend ist festzuhalten, dass die vorliegende Erfindung ein vollsterilisierbares Gerät 101 zur Erzeugung von Strahlenquellen-Abstandshalter-Ketten offenbart. Das Gerät 101 benötigt keine elektromechanischen Bauteile. Die Anordnung der Strahlenquellen und Abstandshalter 115, 117 in den Ketten kann entsprechend eines patientenbezogenen Behandlungsplanes individuell und variabel konfiguriert erfolgen.

Zur Erzeugung der Strahlenquellen-Abstandshalter-Kette benötigt das beschrieben Gerät 101 lediglich zwei Magazine 116, 118, wodurch Verwirrungen bei der Zusammenstellung der Kette minimiert werden. Die Magazine 116, 118 sind in einer Reihe entlang der Gerätelängsachse angeordnet. Dabei entsteht ein für den Nutzer übersichtliches und anwenderfreundliches Erscheinungsbild.

Durch die beschriebene Kodierung von Gerät 101 und Magazin 116, 118 ist es unmöglich, das Strahlenquellen- bzw. das Abstandshalter-Magazin beim Einsatz in das Gerät 101 zu vertauschen.

Beide Magazine 116, 118 transportieren die bevorrateten Implantate in einen unter ihnen angeordneten und an dieser Stelle nach oben hin offenen Arbeitskanal 106. In diesem Kanal 106 werden die Implantate in den vorderen Bereich des Gerätes 101 verschoben, wo sie später zum sogenannten Strand gefügt werden. Dieses Prinzip verhindert, dass die Implantate aus dem hinteren Magazin 118 durch das vordere Magazin 116 geführt werden müssen, wodurch es zum Klemmen kommen könnte. Darüber hinaus verhindert dieses Prinzips Beschädigungen des Mandrels 107, falls ein Magazin 116, 118 versehentlich zu früh aus dem Gesamtaufbau entnommen wird. Der Abwurf der Implantate in nur einen einzigen Arbeitskanal 106 zum Abtransport und Fügen beider Kettenglieder minimiert die Gefahr eines Verbiegens des Mandrels 107.

Aufgrund dieser Anordnung von Magazinen 116, 118 zum Arbeitskanal 106 ist es nicht nötig, die Magazine 116, 118 zum Wechsel des auszuwerfenden Implantats in einem Winkel relativ zur Gerätelängsachse zu verschieben.

Die Magazine enthalten bevorzugt mehr als 50 und bis zu 100 Kettenbestandteilen, die in einer Karussellanordnung platzsparend bevorratet sind. Es können aber auch mehr als 100 Kettenbestandteile in den Magazinen gelagert werden. Wegen des Inhalts von bevorzugt ca. 100 Strahlenquellen bzw. 100 Abstandshaltern werden sich die meisten Strahlenquellenbehandlungen mit nur jeweils einem Magazin 116, 118 durchführen lassen.

Durch Sperren ist das Magazin 116, 118 nicht mehr auslösbar, wenn der Strahlenquellen- bzw. Abstandshalter-Vorrat verbraucht ist.

Der Antrieb der Magazine 116, 118 erfolgt durch die Verwendung einer Konstantkraftfeder.3 zu jeder Zeit mit der gleichen Kraft. Die Hemmung dieser Konstantkraftfeder 3 wird durch ein von außen angetriebenes Ankersystem 4 erzeugt.

Die Magazine 116, 118 sind nach der Behandlung wiederverwendbar. Sie beinhalten keine elektromechanischen Bauteile.

Die Anzahl der in den Magazinen verbliebenen Strahlenquellen und Abstandshaltern 115, 117 ist jederzeit an den Magazinen 116, 118 ablesbar. Daher ist kein Zählen der verbrauchten Strahlenquellen und Abstandshalter 115, 117 während und nach der Behandlung mehr nötig.

Die Magazine 116, 118 bieten aufgrund ihres nach allen Seiten abschirmenden Gehäuses 102 einen optimalen Strahlenschutz. Erst nach dem Einsetzen des Magazins 116, welches die aktiven Strahlenquellen 115 beinhaltet, wird die Öffnung 10 des Strahlenquellenmagazins 116 freigegeben. Bei Entnahme des Magazins 116 wird die Öffnung 10 wieder geschlossen. Der Strahlenschutz ist daher über den gesamten Zeitraum der Anwendung und während des Transportes, d.h. auch außerhalb des Geräts gewährleistet. Bei dem Magazin der nichtaktiven Abstandshalter 117 kann auf dieses Funktionsdetail verzichtet werden.

Die individuell an den Patienten angepasste Strahlenquellen-Abstandshalter-Kettenkonfiguration vermindert im Gegensatz zu vorgefertigten Strahlenquellen-Abstandshalter-Ketten freien Strahlenquellen-Abfall. Nicht verwendete Strahlenquellen 115 und Abstandshalter 117 verbleiben in den entsprechenden Magazinen 116, 118. Eine Strahlenexposition kann daher nicht auftreten.

Darüber hinaus wird eine Strahlenexposition durch die freigesetzten Strahlenquellen im Arbeitskanal 106 durch eine Abschirmung und eine indirekte Draufsicht durch einen Spiegel verhindert.

### Bezugszeichenliste

- 1: Magazin
- 2: Mittel zur Aufnahme von Kettenbestandteilen oder Seedablage
- 3: Erste Spannfeder - Konstantkraftfeder
- 4: Auswerferhebel
- 5: Verschlussschieber
- 6: Vertiefungen
- 7: Magazingehäuse
7a Gehäusedeckel
7b Gehäuseschale
- 8: Innerer Lagerring
- 9: Sicherungsnuten
- 10: Auswurf
- 11: Kodierungsbohrung
- 12: Öffnung
- 13: Oberer Zahnkranz
- 14: Anzeige
- 15: Markierung
- 16: Innere Wand
- 17: Zentrale Öffnung
- 18: Abstandshalter
- 19: Strahlenquellen
- 20: Erste Druckfeder - für den Auswerferhebel 4
- 21: Zweite Druckfeder - für den Verschlussschieber 5
- 22: Blockierelement der Auslösung bei leeren Magazinen

- 101: Gerät zum Fügen von Strahlenquellen
- 102: Gerätegehäuse

- 103: Nadelhalter
- 104: Füge- und Sichteinheit
104a Fügebereich
104b Sichtbereich
- 105: Beladungseinheit
- 106: Arbeitskanal
- 107: Mandrel
- 108: Äußerer Griff
- 109: Spiegel-Linsen-Einheit
109a Spiegel
109b Linse
- 110: Erste Sperre - Sperre im Arbeitskanal zum Fügen der Kettenbestandteile
110a Taster der ersten Sperre
110b Aufnahmeöffnung für erste Verlängerung der Schubstange 113g
- 111: Klappe
- 112: Schlitten
- 113: Hebel-Mechanismus
113a Taster des Hebel-Mechanismus
113b Tasterstange
113c Hebel
113d Erstes Federdruckstück
113e Schubstange
113f Zweites Federdruckstück
113g Erste Verlängerung der Schubstange 113e
113h Zweite Verlängerung der Schubstange 113h
- 114: Magazin
- 115: Strahlenquelle
- 116: Strahlenquellenmagazin

- 117: Abstandshalter
- 118: Abstandshaltermagazin
- 119: Dritte Sperre - Sperre im Griff
119a Taster der dritten Sperre
119b Aufnahmeöffnung für zweite Verlängerung der Schubstange 113h
- 120: Bauraum für Beleuchtung
- 121: Öffnung der Beladungseinheit zum Arbeitskanal
- 122: Magnetkupplung
- 123: Sicherungsmechanismus Magazine
123a Vorsprung des Sicherungsmechanismus 123
123b Taste des Sicherungsmechanismus 123
- 124: Nadel

## Patentansprüche

1. Magazin (1) für Kettenbestandteile einer Kette mit radioaktiven Strahlenquellen (19), umfassend:
ein Gehäuse (7),
ein erstes Mittel zur Aufnahme von Kettenbestandteilen (2), das drehbar in dem Gehäuse (7) gelagert ist und Vertiefungen (6) zur Aufnahme von Kettenbestandteilen aufweist,
einen Auswurf (10) zum Auswurf von Kettenbestandteilen (2),
**dadurch gekennzeichnet, dass**
eine Spannfeder (3) zum Vortrieb des Mittels zur Aufnahme von Kettenbestandteilen (2) in dem Gehäuse (7) angeordnet ist,
ein Zahnkranz (13), der fest mit dem Mittel zur Aufnahme von Kettenbestandteilen (2) gekoppelt ist, drehbar in dem Gehäuse (7) angeordnet ist,
und
ein Auswerferhebel (4) rotatorisch so in dem Gehäuse gelagert ist, dass er in einer ersten Stellung in den Zahnkranz (13) eingreift und in einer zweiten Stellung den Auswurf (10) blockiert.

2. Magazin nach Anspruch 1, ferner umfassend einen Verschlussschieber (5) zum Öffnen und Verschließen des Auswurfs (10), der verschiebbar in dem Gehäuse (7) gelagert ist.

3. Magazin nach Anspruch 1 oder 2, wobei die Vertiefungen (6) auf dem Umfang des Mittels zur Aufnahme von Kettenbestandteilen (2) angeordnet sind und ferner eine Begrenzung (16) um das Mittel zur Aufnahme von Kettenbestandteilen (2) herum im Gehäuse (7) ausgebildet ist, wobei die Größe des Abstandes von Begrenzung (16) und dem Mittel zur Aufnahme von Kettenbestandteilen (2) derart ausgebildet ist, dass Kettenbestandteile zwischen dem Mittel zur Aufnahme von Kettenbestandteilen (2) und der Begrenzung (16) im Inneren des Gehäuses (7) lagerbar und führbar sind.

4. Magazin nach einem der Ansprüche 1 bis 3, wobei die Spannfeder (3) eine Konstantkraftfeder ist.

5. Magazin nach Anspruch 4, wobei das Gehäuse (7) einen kreisförmigen inneren Lagerring (8) aufweist, an dem die Konstantkraftfeder (3) sowie das Mittel zur Aufnahme von Kettenbestandteilen (2) und der darüber liegende Zahnkranz (13) gelagert sind.

6. Magazin nach Anspruch 4 oder 5, wobei die Konstantkraftfeder (3) unter dem Mittel zur Aufnahme von Kettenbestandteilen (2) und der Zahnkranz (13) über dem Mittel zur Aufnahme von Kettenbestandteilen (2) angeordnet ist und/oder wobei das Mittel zur Aufnahme von Kettenbestandteilen (2) und der Zahnkranz (13) einstückig ausgebildet sind.

7. Magazin nach Anspruch 6, wobei der Bereich des Mittels zur Aufnahme von Kettenbestandteilen (2), der am inneren Lagerring (8) des Gehäuses (7) anliegt, von außen betrachtbar ausgebildet ist und mit mindestens einer Markierung (15) zur Inhaltsanzeige versehen ist.

8. Magazin nach einem der Ansprüche 1 bis 7, wobei das Gehäuse umfasst:
Sicherungsnuten (9) zum Eingriff von Sicherungselementen, am Gehäuse angeordnet, und/oder
eine Kodierungsöffnung (11), die mit dem Verschlussschieber (4) derart zusammenwirkt, dass bei Einführung eines entsprechenden Kodierungselementes in die Kodierungsöffnung (11) der Verschlussschieber (4) den Auswurf (10) freigibt.

9. Magazin nach einem der Ansprüche 1 bis 8, wobei das Gehäuse (7) aus einem strahlungsabsorbierbaren Material, bevorzugt Edelstahl, besteht.

10. Magazin nach einem der Ansprüche 2 bis 9, wobei der Auswerferhebel (4) über eine erste Druckfeder (20) und der Verschlussschieber (5) über eine zweite Druckfeder (21) verspannt ist.

11. Magazin nach einem der Ansprüche 2 bis 10, wobei der Auswerferhebel (4) über eine zweite Öffnung (12) im Gehäuse von außen betätigbar ist.

12. Magazin nach einem der Ansprüche 1 bis 11, wobei der Auswurf (10) so angeordnet ist, dass die Kettenbestandteile aus dem Magazin heraus vom Magazin weg ausgeworfen werden (4) und/oder der Auswurf (10) an einer unteren Stirnseite des Magazins (1) angeordnet ist.

13. Magazin nach einem der Ansprüche 1 bis 12, wobei im Gehäuse (7) ein Mittel zum Blockieren des Weiterdrehens des Mittels zur Aufnahme von Kettenbestandteilen (2) nach Auswurf eines Kettenbestandteils realisiert ist.

14. System aus einem Kettenbestandteil für eine Kette mit radioaktiven Strahlenquellen und einem Magazin nach einem der Ansprüche 1 bis 13, wobei ein Mittel zum Blockieren des Weiterdrehens des Mittels zur Aufnahme von Kettenbestandteilen (2) nach Auswurf eines Kettenbestandteils durch ein Zusammenwirken von dem dem ausgeworfenen Kettenbestandteil nachfolgendem Kettenbestandteil und dem Auswerferhebel (4) erfolgt.

15. System nach Anspruch 14, wobei der Auswerferhebel (4) in besagter zweiter Stellung den Auswurf (10) blockiert und der dem Auswurf (10) nächste Kettenbestandteil gegen den Auswerferhebel drückt und so ein Weiterdrehen des Mittels zur Aufnahme von Kettenbestandteilen (2) blockiert.

## Claims

1. A cartridge (1) for chain components of a chain with radioactive radiation sources (19), encompassing:
a housing (7),
a first means for accommodating chain components (2),
which is rotatably mounted in the housing (7), and
exhibits depressions (6) for accommodating chain components,
an ejection port (10) for ejecting chain components (2),
**characterized in that**
a tension spring (3) for advancing the means for accommodating chain components (2) is arranged in the housing (7),
a sprocket (13) rigidly coupled with the means for accommodating chain components (2) is rotatably arranged in the housing (7),
and
an ejector lever (4) is rotatably mounted in the housing in such a way that it engages into the sprocket (13) in a first position, and blocks the ejector port (10) in a second position.

2. The cartridge according to claim 1, further encompassing a sliding gate (5) for opening and closing the ejector port (10), which is slidably mounted in the housing (7).

3. The cartridge according to claim 1 or 2, wherein the depressions (6) are arranged on the periphery of the means for accommodating chain components (2), and a boundary (16) is further formed around the means for accommodating chain components (2) in the housing (7), wherein the distance between the boundary (16) and the means for accommodating chain components (2) is large enough to mount and guide chain components between the means for accommodating chain components (2) and the boundary (16) inside the housing (7).

4. The cartridge according to one of claims 1 to 3, wherein the tension spring (3) is a constant-force spring.

5. The cartridge according to claim 4, wherein the housing (7) exhibits a circular inner bearing ring (8), on which are mounted the constant-force spring (3) as well as the means for accommodating chain components (2) and the sprocket (13) lying over it.

6. The cartridge according to claim 4 or 5, wherein the constant-force spring (3) is arranged under the means for accommodating chain components (2) and the sprocket (13) is arranged over the means for accommodating chain components (2) and/or wherein the means for accommodating chain components (2) and the sprocket (13) are designed as a single piece.

7. The cartridge according to claim 6, wherein the area of the means for accommodating chain components (2) that abuts the inner bearing ring (8) of the housing (7) is designed to be viewed from outside and provided with at least one label (15) to display the contents.

8. The cartridge according to one of claims 1 to 7, wherein the housing encompasses:
Retention grooves (9) for engaging retention elements, arranged on the housing, and/or
a coding opening (11), which interacts with the sliding gate (4) in such a way that the sliding gate (4) releases the ejector port (10) when a corresponding is introduced into the coding opening (11).

9. The cartridge according to one of claims 1 to 8, wherein the housing (7) consists of a radiation-absorbing material, preferably high-grade steel.

10. The cartridge according to one of claims 2 to 9, wherein the ejector lever (4) is braced via a first compression spring (20), and the sliding gate (5) is braced via a second compression spring (21).

11. The cartridge according to one of claims 2 to 10, wherein the ejector lever (4) can be activated from outside by way of a second opening (12) in the housing.

12. The cartridge according to one of claims 1 to 11, wherein the ejector port (10) is arranged in such a way that the chain components are ejected out of the cartridge away from the cartridge (4) and/or the ejector port (10) is situated on a lower face of the cartridge (1).

13. The cartridge according to one of claims 1 to 12, wherein a means for blocking the continued rotation of the means for accommodating chain components (2) after a chain component has been ejected is realized in the housing (7).

14. A system comprised of a chain component for a chain with radioactive radiation sources and a cartridge according to one of claims 1 to 13, wherein a means for blocking the continued rotation of the means for accommodating chain components (2) after a chain component has been ejected involves an interaction between a chain component that follows the ejected chain component and the ejector lever (4).

15. The system according to claim 14, wherein the ejector lever (4), when in the claimed second position, blocks the ejection port (10) and presses the next chain component at the ejection port (10) against the ejector lever, thereby blocking the continued rotation of the means for accommodating chain components (2).

## Revendications

1. Magazine (1) pour les composants de chaîne d'une chaîne avec des sources de rayonnement radioactives (19), comprenant :
un boîtier (7),
un premier moyen pour la réception de composants de chaîne (2), monté de façon rotative dans le boîtier (7) et comportant des renfoncements (6) pour la réception de composants de chaîne,
un dispositif d'éjection (10) pour l'éjection de composants de chaîne (2),
**caractérisé en ce que**
un ressort de précontrainte (3) pour l'avancement du moyen pour la réception de composants de chaîne (2) est installé dans le boîtier (7),
une couronne dentée (13) accouplé fixement avec le moyen pour la réception de composants de chaîne (2) est installée de façon rotative dans le boîtier (7),
et
un levier d'éjection (4) est monté de telle façon en rotation dans le boîtier, qu'il s'engage dans la couronne dentée (13) dans une première position et bloque le dispositif d'éjection (10) dans une deuxième position.

2. Magazine selon la revendication 1, comprenant en outre un poussoir de fermeture (5) pour l'ouverture et la fermeture du dispositif d'éjection (10), installé de façon coulissante dans le boîtier (7).

3. Magazine selon la revendication 1 ou 2, dans lequel les renfoncements (6) sont disposés sur le pourtour du moyen pour la réception de composants de chaîne, et une délimitation (16) entourant le moyen pour la réception de composants de chaîne est formée dans le boîtier (7), dans lequel la dimension de l'écart entre la délimitation (16) et le moyen pour la réception de composants de chaîne (2) est conçue de telle manière que les composants de chaîne entre le moyen pour la réception de composants de chaîne (2) et la délimitation (16) peuvent être stockés et guidés à l'intérieur du boîtier (7).

4. Magazine selon l'une des revendications 1 à 3, dans lequel le ressort de précontrainte (3) est un ressort à force constante.

5. Magazine selon la revendication 4, dans lequel le boîtier (7) comporte une bague de roulement interne circulaire (8), sur laquelle sont montés le ressort à force constante (3) ainsi que le moyen pour la réception de composants de chaîne (2) et la couronne dentée (13) située au-dessus.

6. Magazine selon la revendication 4 ou 5, dans lequel le ressort à force constante (3) est monté sous le moyen pour la réception de composants de chaîne (2) et la couronne dentée (13) est montée au-dessus du moyen pour la réception de composants de chaîne (2), et/ou dans lequel le moyen pour la réception de composants de chaîne (2) et la couronne dentée (13) sont conçus d'une seule pièce.

7. Magazine selon la revendication 6, dans lequel la région du moyen pour la réception de composants de chaîne (2) qui s'applique sur la bague de roulement interne (8) du boîtier (7) est visible de l'extérieur et pourvue d'au moins un repère (15) pour l'indication du contenu.

8. Magazine selon l'une des revendications 1 à 7, dans lequel le boîtier comprend :
des rainures de blocage (9) pour l'engagement d'éléments de blocage, disposées sur le boîtier, et/ou une ouverture de codage (11) coopérant de telle manière avec le poussoir de fermeture (4) que lors de l'insertion de l'élément de codage correspondant dans l'ouverture de codage (11), le poussoir de fermeture (4) libère de dispositif d'éjection (10).

9. Magazine selon l'une des revendications 1 à 8, dans lequel le boîtier (7) est constitué d'un matériau apte à absorber le rayonnement, de préférence de l'acier fin.

10. Magazine selon l'une des revendications 2 à 9, dans lequel le levier d'éjection (4) est bloqué par un premier ressort de pression (20) et le poussoir de fermeture (5) est bloqué par un deuxième ressort de pression (21).

11. Magazine selon l'une des revendications 2 à 10, dans lequel le levier d'éjection (4) peut être actionné de l'extérieur par une deuxième ouverture (12) dans le boîtier.

12. Magazine selon l'une des revendications 1 à 11, dans lequel le dispositif d'éjection (10) est disposé de telle manière que les composants de chaîne dans le magazine sont éjectés (4) hors du magazine, et/ou le dispositif d'éjection (10) est disposé sur une face frontale inférieure du magazine (1).

13. Magazine selon l'une des revendications 1 à 12, dans lequel un moyen pour le blocage de la rotation supplémentaire du moyen pour la réception de composants de chaîne (2) après l'éjection d'un composant de chaîne est réalisé dans le boîtier (7).

14. Système constitué d'un composant de chaîne pour une chaîne avec des sources de rayonnement radioactives et d'un magazine selon l'une des revendications 1 à 13, dans lequel un moyen pour le blocage de la rotation supplémentaire du moyen pour la réception de composants de chaîne (2) après l'éjection d'un composant de chaîne est réalisé par la coopération entre le composant de chaîne consécutif au composant de chaîne éjecté et le levier d'éjection (4).

15. Système selon la revendication 14, dans lequel le levier d'éjection (4) bloque le dispositif d'éjection (10) dans ladite deuxième position, et le composant de chaîne le plus proche du dispositif d'éjection (10) appuie sur le levier d'éjection, bloquant ainsi une rotation supplémentaire du moyen pour la réception de composants de chaîne (2).
